# EUROPEAN PATENT APPLICATION

(11) **EP 4 509 462 A1**
(43) Date of publication of application: **19.02.2025**
(21) Application number: 23788432.5
(22) Date of filing: 14.04.2023
(51) Int. Cl.: C01B 25/08, B01J 27/185, B01J 35/02, B01J 37/04, B82Y 30/00, B82Y 40/00, C07B 61/00, C07C 209/48, C07C 211/07, C07C 211/09, C07C 211/17, C07C 211/19, C07C 211/27, C07C 211/29, C07C 211/30, C07C 217/58, C07C 321/28

(54) **IRON PHOSPHIDE NANOPARTICLES, AND COMPOSITE BODY AND REDUCTION CATALYST EACH CONTAINING SAME**

(30) Priority: 15.04.2022 JP 2022067961
(71) Applicant: OSAKA UNIVERSITY, Suita-shi Osaka 565-0871 (JP)
(72) Inventor: MITSUDOME, Takato, Suita-shi, Osaka 565-0871 (JP); TSUDA, Tomohiro, Suita-shi, Osaka 565-0871 (JP)
(74) Representative: D Young & Co LLP
(86) International application number: PCT/JP2023/015245
(87) International publication number: WO 2023/200015

(57) **Abstract**

The present invention provides iron phosphide nanoparticles in which iron atoms are in a low valence state and which are stable under an atmospheric condition, a production method therefor, and a reduction catalyst. The present invention relates to iron phosphide nanoparticles having peaks at diffraction angles (2θ±0.5°) of 48.3° and 32.7° in a powder X-ray diffraction measurement using CuKα radiation, wherein, when the iron phosphide nanoparticles are measured by X-ray photoelectron spectroscopy (XPS), iron atoms contained therein have a peak in a range of 706.0 to 707.5 eV in an Fe2p_{3/2} spectrum.

## Description

### TECHNICAL FIELD

The present invention relates to iron phosphide nanoparticles, and a composite body and a reduction catalyst each containing the same.

### BACKGROUND ART

Iron is one of the most reliable metals for the future in terms of cost and toxicity as well. Iron nanoparticles having a low valence (0 valence) are a catalyst material extremely effective for reactions essential to human society, such as the Harber-Bosch process in which ammonia is synthesized from nitrogen, and the Fischer-Tropsch reaction in which gasoline is synthesized from carbon monoxide.

However, low-valent iron nanoparticles are extremely unstable, and thus, are easily oxidized by oxygen at a ppm order, to be inactivated. That is, unstable iron nanoparticles need to be handled in a strict anaerobic atmosphere. Therefore, the method is limited to in-situ reduction of iron ions supported by a carrier under a high temperature-high hydrogen pressure condition.

The catalytic activity of conventionally known general nanoparticles largely depends on, due to unstableness thereof, the size and shape of the particles, the type of the carrier, and the additive. However, in the above preparation method, precise control of the size of the nanoparticles is difficult, and further, the carrier and the additive are limited to materials that can withstand high temperature.

Therefore, although iron nanoparticles are an extremely attractive catalyst material, unstableness of the iron nanoparticles largely hinders the progress of catalytic chemistry thereof. In actuality, as for the iron catalyst used in the Harber-Bosch process, the catalyst has not been significantly improved even now after more than 100 years after the development.

Therefore, there is a need for an unconventional innovative technology that controls the conflicting characteristics, i.e., development of iron nanoparticles in which iron atoms are in a low valence state and which are stable under an atmospheric condition.

Meanwhile, a production method for nano-sized metal particles has also been proposed (e.g., Non Patent Literature 1).

### CITATION LIST

### Non Patent Literature

Non Patent Literature 1: "Triphenyl Phosphite as the Phosphorus Source for the Scalable and Cost-Effective Production of Transition Metal Phosphides", Chemistry of Materials, Junfeng Liu et al., 2018, 30, pp 1799-1807

### SUMMARY OF INVENTION

### Technical Problem

However, Non Patent Literature 1 does not indicate that the obtained nano-sized metal particles are in a low valence state and are stable under an atmospheric condition. In addition, Non Patent Literature 1 does not suggest use of the obtained nano-sized metal particles as a reduction catalyst, either.

Further, it was found that, with the production method according to Non Patent Literature 1, it is difficult to produce, at an industrially usable level, a reduction catalyst that can be used in a reduction reaction such as hydrogenation of an organic compound such as a nitrile compound.

An object of the present invention is to provide iron phosphide nanoparticles in which iron atoms are in a low valence state and which are stable under an atmospheric condition, a production method therefor, and a reduction catalyst.

### Solution to Problem

The present inventors conducted thorough studies in order to solve the above problem, found that the above problem can be solved by alloying iron and phosphorus and using iron phosphide nanoparticles, then, further advanced their studies, based on this finding, and completed the present invention.

The present invention encompasses the following invention.
[1] Iron phosphide nanoparticles having peaks at diffraction angles (2θ±0.5°) of 48.3° and 32.7° in a powder X-ray diffraction measurement using CuKα radiation, wherein
   when the iron phosphide nanoparticles are measured by X-ray photoelectron spectroscopy (XPS), iron atoms contained therein have a peak in a range of 706.0 to 707.5 eV in an Fe2p_{3/2} spectrum.
[2] The iron phosphide nanoparticles according to [1], further having peaks at 40.2°, 52.9°, and 54.6° in the powder X-ray diffraction measurement.
[3] The iron phosphide nanoparticles according to [1] or [2], further having a peak at 46.3° in the powder X-ray diffraction measurement.
[4] The iron phosphide nanoparticles according to [1] or [2], wherein the iron phosphide nanoparticles are each a rod-shaped particle, and a maximum length in a major axis direction of the rod-shaped particle is less than 100 nm.
[5] The iron phosphide nanoparticles according to [1] or [2], wherein a presence ratio between phosphorus atoms and iron atoms according to a scanning transmission electron microscope (STEM)-energy-dispersive X-ray spectroscopy (EDX) composition analysis is P:Fe=20%:80% to 80%:20%.
[6] A composite body containing the iron phosphide nanoparticles according to [1] or [2] and a carrier.
[7] The composite body according to [6], wherein the carrier is at least one type selected from the group consisting of a polymer, a chalcogen compound, a metal compound, a metal, and a solid carbon material.
[8] A reduction catalyst containing the iron phosphide nanoparticles according to [1] or [2].
[9] The reduction catalyst according to [8], further containing a carrier, wherein the iron phosphide nanoparticles and the carrier form a composite body.
[10] A production method for a hydrogenated organic compound, wherein an organic compound is hydrogenated by using the reduction catalyst according to [8], to obtain the hydrogenated organic compound.
[11] The production method for the hydrogenated organic compound according to [10], wherein the organic compound is a nitrile compound, the hydrogenated organic compound is a primary amine compound, and the nitrile compound is hydrogenated in a hydrogen atmosphere and in the presence of ammonia, at a hydrogen pressure of 8 MPa or less.
[12] A production method for the iron phosphide nanoparticles according to [1] or [2], the production method comprising:
   mixing a phosphorus compound and a surfactant under heating;
   further heating an obtained mixture; and
   further mixing an iron carbonyl compound thereto and heating a resultant matter, wherein
   1-octadecene is not used.
[13] The production method for the iron phosphide nanoparticles according to [12], wherein the phosphorus compound is a phosphorous acid ester compound.
[14] The production method for the iron phosphide nanoparticles according to [12], wherein the surfactant is an alkylamine.
[15] The production method for the iron phosphide nanoparticles according to [12], wherein the iron carbonyl compound is at least one type selected from the group consisting of Fe(CO)s, Fe₂(CO)₉, and Fe₃(CO)₁₂.

### Advantageous Effects of Invention

The present invention can provide iron phosphide nanoparticles in which iron atoms are in a low valence state and which are stable under an atmospheric condition, a production method therefor, and a reduction catalyst.

With the iron phosphide nanoparticles of the present invention, when used as a reduction catalyst (e.g., hydrogenation catalyst, or the like), it is possible to perform a reduction reaction (e.g., hydrogenation reaction) under a gentler condition as compared with a conventional technology such as the Harber-Bosch process, to produce a target compound.

The iron phosphide nanoparticles and the reduction catalyst of the present invention use iron, which is present also in a living body, as the metal source, and thus has low toxicity and also is excellent in safety. Further, from the abundance of the existence amount (reserves) of iron present on Earth, industrial advantages also in terms of cost are provided.

### BRIEF DESCRIPTION OF THE DRAWINGS

FIG. 1 shows images of transmission electron microscope (TEM) observation of iron phosphide nanoparticles and a composite body containing the same according to an embodiment of the present invention.
FIG. 2A shows a measurement result of a powder X-ray diffraction measurement on the iron phosphide nanoparticles according to an embodiment of the present invention.
FIG. 2B shows peak positions of crystal planes corresponding to FeP and Fe₂P in addition to the measurement result in FIG. 2A.
FIG. 3 shows a measurement result according to XPS on iron phosphide nanoparticles according to Example 1-1.
FIG. 4A shows a measurement result of XANES regarding the K absorption edge of Fe in the iron phosphide nanoparticles and the composite body.
FIG. 4B shows a measurement result of FT-EXAFS.
FIG. 5 shows a result of an XAFS measurement on the iron phosphide nanoparticles according to Example 1-1. In the partially enlarged view in FIG. 5, the uppermost line represents room temperature and the lowermost line represents 700°C.
FIG. 6 shows TEM images before and after use of a composite body according to Example 3-2 as a catalyst.
FIG. 7 shows a result of a durability test on the composite body according to Example 3-2 as a catalyst.

### DESCRIPTION OF EMBODIMENTS

Hereinafter, embodiments of the present invention will be described. In the present description, the upper limit value and the lower limit value of a numerical value range (the size, etc., of iron phosphide nanoparticles, the use amount of a certain component (material of a compound, etc.), the temperature, the pressure, or a value calculated from each numerical value range, each physical property, etc.) can be combined as appropriate.

A certain embodiment of the present invention relates to iron phosphide nanoparticles. Hereinafter, description will be given for each embodiment. It should be noted that the present invention is not limited by the embodiments below.

Iron phosphide nanoparticles of the present invention have peaks at diffraction angles (2θ±0.5°) of 48.3° and 32.7° in a powder X-ray diffraction measurement using CuKα radiation, and
when the iron phosphide nanoparticles are measured by X-ray photoelectron spectroscopy (XPS), iron atoms contained therein have a peak in a range of 706.0 to 707.5 eV in an Fe2p_{3/2} spectrum.

In the iron phosphide nanoparticles of the present invention, iron atoms are in a low valence state (metallic state), and the iron phosphide nanoparticles are excellent in stability under an atmospheric condition. "Valence" is a number representing how many atoms of other elements an atom of a certain element can bind to. Iron (Fe) atoms contained in the iron phosphide nanoparticles of the present invention are in a low valence state.

The iron phosphide nanoparticles of the present invention have peaks at diffraction angles (2θ±0.5°) of 48.3° and 32.7° in a powder X-ray diffraction measurement using CuKα radiation. The diffraction angle may be 2θ±0.2° or may be 2θ±0.5°.

In the powder X-ray diffraction analysis (XRD) using CuKα radiation, a known X-ray diffractometer can be used. As the X-ray diffractometer, a commercial product (e.g., a fully automatic multipurpose X-ray diffraction apparatus (trade name "Philips X'PERT MPD diffractometer" manufactured by Philips Japan Ltd.)) can be used.

The iron phosphide nanoparticles of the present invention are in a low valence state and have a property (hereinafter, also referred to as "atmospheric stability") that can stably maintain the structure of the compound under an atmospheric condition. Therefore, under a condition of presence of oxygen, the crystal structure can be measured through an X-ray diffraction analysis.

In the present description, "under an atmospheric condition" means having an oxygen concentration at about the oxygen concentration (about 21%) in the atmosphere. The iron phosphide nanoparticles of the present invention are excellent in stability under a condition of presence of oxygen. As for the condition of presence of oxygen (O₂), the concentration of oxygen is not particularly limited, and may be about the oxygen concentration (about 21%) in the atmosphere, and may be about 22% to 100%. The iron phosphide nanoparticles of the present invention are excellent in atmospheric stability, and can maintain a low valence state under the condition of presence of oxygen irrespective of the oxygen concentration.

Preferably, the iron phosphide nanoparticles of the present invention further have a peak at 46.3° in the powder X-ray diffraction measurement.

The peaks that the iron phosphide nanoparticles of the present invention have at diffraction angles of 32.7°, 46.3°, and 48.3° in the X-ray diffraction pattern (hereinafter, also referred to as "XRD pattern") using CuKα radiation respectively correspond to crystal planes of the (011) plane, the (112) plane, and the (211) plane of FeP.

As a certain preferable embodiment, the iron phosphide nanoparticles of the present invention are exemplified by iron phosphide nanoparticles composed of FeP being in a low valence state and having atmospheric stability.

As another preferable embodiment, iron phosphide nanoparticles that further have peaks at 40.2°, 52.9°, and 54.6° in the powder X-ray diffraction measurement are exemplified.

The peaks that the iron phosphide nanoparticles of the present invention have at diffraction angles of 40.2°, 52.9°, and 54.6° in the XRD pattern due to CuKα radiation respectively correspond to the (111) plane, the (002) plane, and the (300) plane of Fe₂P.

That Fe₂P and FeP can be identified by the peaks at the diffraction angles is also clear from the JCPDS card (File 85-1727 and 39-0809) of a database (Powder Diffraction File, level 4 plus) of the International Centre for Diffraction Data (ICDD).

In the present description, the iron phosphide nanoparticles of the present invention are also collectively referred to as "nano-FeₓP (x represents 1 or 2).

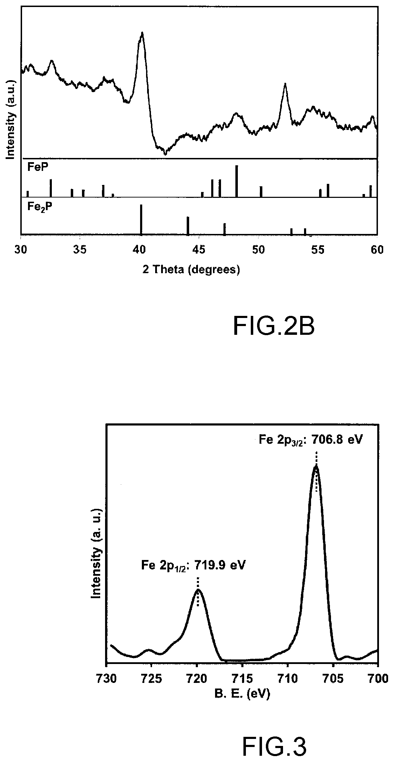

In the iron phosphide nanoparticles of the present invention, iron atoms contained therein can maintain a low valence state without being oxidized in the atmosphere. That the iron atoms contained in the iron phosphide nanoparticles of the present invention have a low valence can be confirmed by a measurement according to X-ray photoelectron spectroscopy (XPS), for example. As a measurement result according to the XPS, a result as shown in FIG. 3 is obtained. In FIG. 3, the vertical axis represents photoelectron intensity and the horizontal axis represents binding energy (unit: eV).
"Iron atoms have a low valence" means that, in an Fe2p_{3/2} spectrum according to X-ray photoelectron spectroscopy (XPS), iron atoms contained in the iron phosphide nanoparticles have a peak in a range of 706.0 to 707.5 eV. The Fe2p_{3/2} spectrum can be measured by a method described in the XPS analysis in Examples described later.

For the XPS, a known X-ray photoelectron spectroscopic analyzer can be used. The X-ray photoelectron spectroscopic analyzer may be a commercial product (e.g., X-ray photoelectron analyzer (trade name "KRATOS ULTRA2", SHIMADZU CORPORATION), a photoelectron spectroscopic apparatus (model number "JPS-9030", manufactured by JEOL Ltd.), a scanning X-ray photoelectron spectroscopic analyzer (model number "PHI Quantera II", ULVAC-PHI, INC.), or the like). As an excitation source, AlKα radiation, MgKα radiation, AgLα radiation, or the like can be used.

With respect to the iron phosphide nanoparticles of the present invention, iron atoms contained in the iron phosphide nanoparticles are in a low valence state, and the iron phosphide nanoparticles are stable under an atmospheric condition. That is, the iron atoms forming the iron phosphide nanoparticles of the present invention can continue to be present in a low valence state in which the iron atoms have an activity as a catalyst, without the state changed over time, under an atmospheric condition. In this regard, the present invention is different from the conventional technology.

The valence of an iron atom in the iron phosphide nanoparticles of the present invention can be analyzed, based on an X-ray absorption fine structure (XAFS), for example. Specifically, a high intensity X-ray, preferably a high intensity X-ray whose energy is continuously changed, is applied to a metal atom, thereby exciting a core electron of the metal atom to an energy level of an unoccupied orbital or more, whereby the excited metal atom releases a photoelectron having a kinetic energy corresponding to the difference between the excitation energy of the incident X-ray and the binding energy of the core electron, and accordingly, a fine structure appears near the absorption edge in the X-ray absorption spectrum of the metal atom. By analyzing this, the electronic state of the metal atom can be identified. In the energy region of such an XAFS, a fine structure appearing at about several tens of eV near the absorption edge is referred to as an X-ray absorption near edge structure (XANES). Meanwhile, in the energy region of the XAFS, a modulated structure continuous from the absorption edge to about the 1000-eV high energy side is referred to as an extended X-ray absorption fine structure (EXAFS). The EXAFS is a vibrational structure resulting from interaction between an excited electron and a scattered electron from a near-neighbor atom, and a radial distribution function obtained through Fourier transform includes information about the local structure of the metal atom (surrounding atomic species, the number of coordinating atoms, interatomic distance).

In the iron phosphide nanoparticles of the present invention, that iron atoms contained therein are present in a low valence state can also be confirmed from the X-ray absorption near edge structure (XANES).

In the iron phosphide nanoparticles of the present invention, iron atoms contained therein are present in a low valence state. Thus, in an XANES spectrum obtained through an XANES measurement regarding the K absorption edge of Fe atoms, rise of the peak is the same as the rise of the peak of iron foil (Fe foil). In other words, that iron atoms forming the iron phosphide nanoparticles of the present invention are present in a low valence state can be confirmed by the fact that, in the XANES spectrum obtained through the XANES measurement, rise of the peak is the same as the rise of the peak of iron foil (Fe foil). The iron foil (Fe foil) is Fe of 0 valence as a metal. An example of a certain preferable embodiment is iron phosphide nanoparticles in which, in an XANES spectrum obtained through an XANES measurement regarding the K absorption edge of Fe atoms, the ratio of (spectrum intensity at 7110 eV of nano-FeₓP) / (spectrum intensity at 7110 eV of Fe foil) is 0.910 to 1.000. "eV" represents binding energy.

In the iron phosphide nanoparticles of the preferable embodiment, the ratio of (spectrum intensity at 7110 eV of nano-FeₓP) / (spectrum intensity at 7110 eV of Fe foil) is preferably 0.930 to 1.000, more preferably 0.950 to 1.000, and further preferably 0.970 to 1.000. The measurement method for the XANES spectrum is described in Examples described later.

In the iron phosphide nanoparticles of the preferable embodiment, further, the ratio of (spectrum intensity at 7111 eV of nano-FeₓP) / (spectrum intensity at 7111 eV of Fe foil) is preferably 0.930 to 1.000, more preferably 0.950 to 1.000, and further preferably 0.970 to 1.000. The measurement method for the XANES spectrum is described in Examples described later.

In the iron phosphide nanoparticles of the preferable embodiment, at each of two points of the spectrum intensity at 7110 eV and the spectrum intensity at 7111 eV, the ratio of (spectrum intensity of nano-FeₓP) / (spectrum intensity of Fe foil) is preferably 0.930 to 1.000, more preferably 0.950 to 1.000, and further preferably 0.970 to 1.000. At the two points, when the ratios between the spectrum intensity of the nano-FeₓP and the spectrum intensity of the Fe foil are approximately the same, it can be said that the rises of the peaks of the iron phosphide nanoparticles and the Fe foil are the same. That the rises of the peaks of the iron phosphide nanoparticles and the Fe foil are the same with each other means that the iron phosphide nanoparticles are in a low valence state.

From the measurement result according to the XPS, it can be said that the surface side of the iron phosphide nanoparticles maintains a low valence state, and since the measurement result of the XANES matches the measurement result according to the XPS, it can also be said that correctness of both measurement results is supported by each other. Thus, it can be said that, not only at the surface of the iron phosphide nanoparticles but also in the entirety of the iron phosphide nanoparticles, the iron atoms contained therein maintain a low valence state.

For the XANES measurement regarding the K absorption edge of Fe atoms, a known measurement apparatus (e.g., an X-ray absorption spectroscopic apparatus (apparatus name "QuantumLeap H2000" (for measurement in the atmosphere), "QuantumLeap V210" (for measurement in vacuum), which are manufactured by Canon Inc.), known measurement equipment (large synchrotron radiation facility "SPring-8": beam line BL01B1, BL14B2, zip code 679-5198, 1-1, Kouto, Sayo-cho, Sayo-gun, Hyogo Prefecture), or the like can be used.

In the Fe2p spectrum in the XPS, Fe atoms having a low valence have a peak in a range of 706.7 to 707.0 eV. This is shown in Fe 2p in Appendix B. Chemical States Tables of Handbook of X-ray Photoelectron Spectroscopy: A Reference Book of Standard Spectra for Identification and Interpretation of XPS Data (Eds.: J. Chastain), Published by Perkin-Elmer Corporation, Electronics Division, Eden Prairie, MN, 1992.

As a certain preferable embodiment, iron phosphide nanoparticles in which, in an Fe2p_{3/2} spectrum according to X-ray photoelectron spectroscopy (XPS), iron atoms contained in the iron phosphide nanoparticles have a peak in a range of 706.0 to 707.5 eV and the value (ratio) of (spectrum intensity at 7110 eV of nano-FeₓP) / (spectrum intensity at 7110 eV of Fe foil) in an XANES spectrum obtained through an XANES measurement regarding the K absorption edge of Fe atoms is 0.910 to 1.000, are exemplified. The ratio of the spectrum intensity in the XANES spectrum is preferably 0.930 to 1.000, more preferably 0.950 to 1.000, and further preferably 0.970 to 1.000.

In the preferable embodiment, further, the ratio of (spectrum intensity at 7111 eV of nano-FeₓP) / (spectrum intensity at 7111 eV of Fe foil) is preferably 0.930 to 1.000, more preferably 0.950 to 1.000, and further preferably 0.970 to 1.000.

Further, in the iron phosphide nanoparticles of the preferable embodiment, at two points of the spectrum intensity at 7110 eV and the spectrum intensity at 7111 eV, the ratio of (spectrum intensity of nano-FeₓP) / (spectrum intensity of Fe foil) is preferably 0.930 to 1.000, more preferably 0.950 to 1.000, and further preferably 0.970 to 1.000.

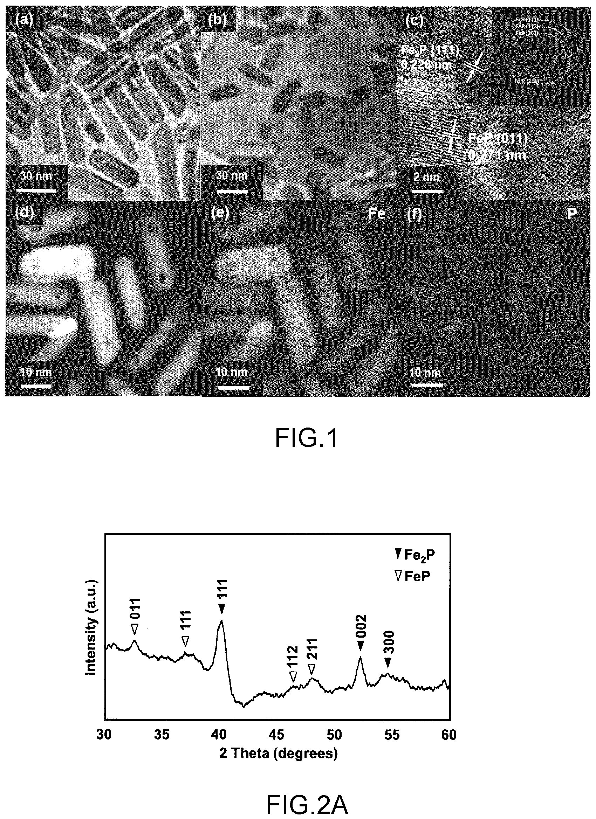

The shape of the iron phosphide nanoparticles of the present invention is not particularly limited, but preferably, the iron phosphide nanoparticles are each a rod-shaped particle. As for the shape of the rod-shaped particle, it is sufficient that the major axis and the minor axis can be recognized, and those not having a strict rod shape are also included. As for the rod-shaped particle, the morphology of the particle can be confirmed through TEM observation as shown in FIG. 1, for example. FIG. 1(a) shows a TEM image of the iron phosphide nanoparticles of the present invention.

In the rod-shaped particle, the length (the maximum length in the major axis direction) in the major axis direction is preferably less than 100 nm, more preferably 90 nm or less, and further preferably 80 nm or less. Depending on the embodiment, the length may be 70 nm or less, 60 nm or less, 50 nm or less, or 40 nm or less. When the length is in the range, the rod-shaped particles can be, in the atmosphere, stably present in a low valence state, the surface area of a single particle can be made larger, and the catalytic activity can be more enhanced. In a certain embodiment, for example, the maximum length of the major axis of the rod-shaped particle is preferably 10 nm or more, more preferably 15 nm or more, and further preferably 20 nm or more.

The length (the maximum length in the major axis direction) in the major axis direction of the rod-shaped particle means an arithmetic average value of the length (the maximum length in the major axis direction) in the major axis direction in 100 arbitrary particles observed in electron microscopic observation. The number of particles to be measured may be set to 200 or 500 as necessary.

When the iron phosphide nanoparticle of the present invention is a rod-shaped particle, the cross-sectional shape at the face (bottom face) having the minor axis is not particularly limited, and may be a hexagon, for example. The shape of the bottom face is not particularly limited, and may be a flat surface, may have protrusions and recesses, or may have a protrusion only.

In the rod-shaped particle, the length (the maximum length in the minor axis direction) in the minor axis direction is not particularly limited as long as the length is shorter than the length (the maximum length in the major axis direction) in the major axis direction. For example, when the cross-sectional shape at the face (bottom face) having the minor axis is a hexagon, as for the length (maximum length) in the minor axis direction, the length of a diagonal having the maximum length in the cross section is defined as the length in the minor axis direction. The length (maximum length) in the minor axis direction is preferably 50 nm or less, more preferably 40 nm or less, and further preferably 30 nm or less. Depending on the embodiment, the length may be 20 nm or less or 10 nm or less.

In the atmosphere, in order to enhance the catalytic activity by increasing the surface area of each particle while the particle can be stably present in a low valence state, the length (maximum length) in the minor axis direction is preferably longer, and may be 1 nm or more, may be 5 nm or more, or may be 9 nm or more.

The length (maximum length) in the minor axis direction means the arithmetic average value of the length of a diagonal having the maximum length in a cross section at the face (bottom face) having the minor axis in 100 arbitrary particles observed in electron microscopic observation. The number of particles to be measured may be set to 200 or 500 as necessary.

In the rod-shaped particle, the aspect ratio (the length in the major axis direction: the length in the minor axis direction) is not particularly limited, but is preferably 100:1 to 100:95, more preferably 100:5 to 100:75, and further preferably 100:10 to 100:50.

The aspect ratio can be calculated from values calculated from the length in the major axis direction and the length in the minor axis direction of the rod-shaped particle.

The size of the iron phosphide nanoparticles of the present invention can be adjusted by selection of the raw material compound in a later-described production method for the iron phosphide nanoparticles and adjustment of concentration, heating temperature, heating condition (agitation speed, etc.), reaction time, and the like.

In the iron phosphide nanoparticles of the present invention, the presence ratio between phosphorus atoms and iron atoms according to a scanning transmission electron microscope (STEM)-energy-dispersive X-ray spectroscopy (EDX) composition analysis is preferably P:Fe=20%:80% to 80%:20%, and from the viewpoint of more enhancement of the catalytic activity and more excellence in atmospheric stability, is more preferably 30%:70% to 70%:30% and further preferably 35%:65% to 65%:35%.

The presence ratio between phosphorus atoms and iron atoms in the iron phosphide nanoparticles of the present invention can be adjusted by adjustment of the degree of phosphorization of iron. Therefore, adjustment such as, in the nano-FeₓP, increasing the compound whose x is 1, or increasing the compound whose x is 2 is possible. In a certain embodiment, in the nano-FeₓP, x is 1 or 2. The iron phosphide nanoparticles contain at least one of FeP and Fe₂P, and those containing both are preferable. In another certain embodiment, in the nano-FeₓP, x is 1. In another embodiment, in the nano-FeₓP, x is 2.

As an adjustment method for the degree of phosphorization of iron, for example, if the heating temperature, etc., in the production method for the iron phosphide nanoparticles is adjusted (e.g., the heating temperature is increased by 10°C, the reaction time is extended by 1 hour, etc.), adjustment of the degree of phosphorization, such as promoting phosphorization of iron, can be realized. Conversely, when phosphorization is inhibited and advancement of phosphorization is stopped at a certain degree, if the conditions such as heating temperature are adjusted in an inversed manner (e.g., the heating temperature is decreased by 10°C, the reaction time is shortened by 1 hour, etc.), the presence ratio between phosphorus atoms and iron atoms can be adjusted.

In the iron phosphide nanoparticles of the present invention, the presence ratio between phosphorus atoms and iron atoms can be evaluated by an elementary analysis method (element mapping). For the elementary analysis method (element mapping), a known energy-dispersive X-ray analysis (EDX) can be used. For the EDX, a known measurement apparatus can be used. As the measurement apparatus, a commercial product (a transmission electron microscope (a single atom analysis transmission electron microscope, trade name "Titan Cubed G2 60-300", acceleration voltage: 300 kV, manufactured by FEI Company (now: Thermo Fisher Scientific (US)), or the like) that includes a super-X energy-dispersive X-ray spectroscopy (EDX) detector, can be used.

The EDX is a method for observing a single particle. However, the presence ratio between phosphorus atoms and iron atoms may be evaluated by observing the entirety of a plurality of (a large amount of) iron phosphide nanoparticles as observed in microscopy. An example of such a method is ICP atomic emission spectroscopy (inductively coupled plasma atomic emission spectroscopy: ICP-AES). For the ICP-AES, a known measurement apparatus can be used. As the measurement apparatus, a commercial product (an ICP atomic emission spectroscopic analyzer, trade name "Optima 8300", manufactured by PerkinElmer, Inc., or the like) can be used. Evaluation by the ICP-AES of the presence ratio between phosphorus atoms and iron atoms can be performed, for example, by a method shown in Examples described later.

The presence ratio between phosphorus atoms and iron atoms evaluated by the ICP-AES is preferably P:Fe=20%:80% to 80%:20%, and from the viewpoint of more enhancement of the catalytic activity or more excellence in atmospheric stability, is more preferably 30%:70% to 70%:30% and further preferably 35%:65% to 65%:35%.

In a certain embodiment, in the iron phosphide nanoparticles of the present invention, with respect to the presence ratio between phosphorus atoms and iron atoms, the ratio evaluated by the ICP-AES (e.g., sequential type) and the ratio evaluated by the EDX are in ranges close to each other. That both values are close to each other means that surplus phosphorus atoms are not attached to the iron phosphide nanoparticles of the present invention.

The iron phosphide nanoparticles of the present invention are excellent not only in atmospheric stability but also in thermal stability since the iron phosphide nanoparticles can maintain the structure thereof also when heated. That the iron phosphide nanoparticles can maintain the structure thereof means that the iron phosphide nanoparticles can maintain a low valence state. Whether or not the structure can be maintained when the iron phosphide nanoparticles are heated can be confirmed by an X-ray absorption fine structure (XAFS) measurement under an atmospheric condition. For example, this can be evaluated by a method shown in Examples described later.

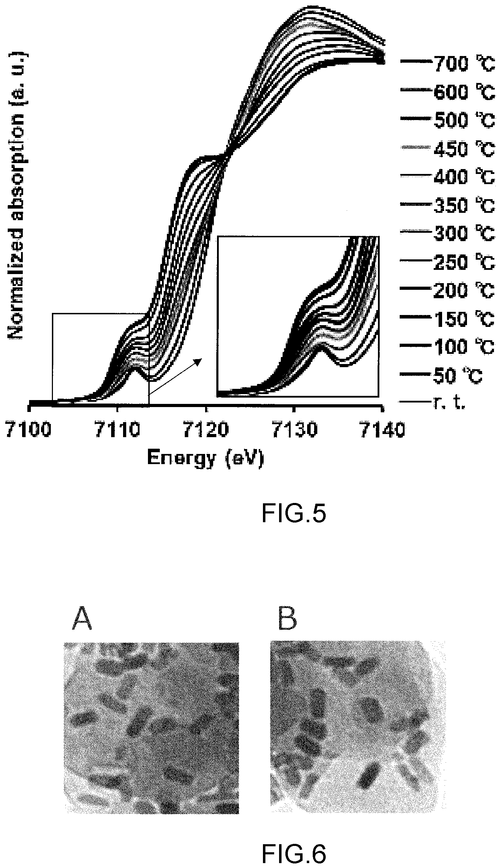

Since the iron phosphide nanoparticles of the present invention are also excellent in thermal stability, when the XAFS measurement is performed by raising the temperature as shown in FIG. 5, the rise of the peak in the XAFS spectrum obtained through measurement under a condition of a temperature increased to 150°C is the same as the rise of the peak at room temperature, for example.

In a certain preferable embodiment, iron phosphide nanoparticles in which, in an XAFS spectrum obtained through an XAFS measurement regarding the K absorption edge of Fe atoms, the ratio of (spectrum intensity at 7110 eV at 100°C) / (spectrum intensity at 7110 eV at room temperature) is 0.910 to 1.000, are exemplified.

"eV" represents binding energy. The ratio is preferably 0.930 to 1.000, more preferably 0.950 to 1.000, and further preferably 0.970 to 1.000.

As another embodiment, iron phosphide nanoparticles in which, in an XAFS spectrum obtained through an XAFS measurement regarding the K absorption edge of Fe atoms, the ratio of (spectrum intensity at 7110 eV at 150°C) / (spectrum intensity at 7110 eV at room temperature) is 0.910 to 1.000, are exemplified. The ratio is preferably 0.930 to 1.000, more preferably 0.950 to 1.000, and further preferably 0.970 to 1.000.

In the iron phosphide nanoparticles of the preferable embodiment, at two points of the spectrum intensity at 7110 eV and the spectrum intensity at 7111 eV at 100°C, the ratio of (spectrum intensity at 100°C) / (spectrum intensity at room temperature) is preferably 0.930 to 1.000, more preferably 0.950 to 1.000, and further preferably 0.970 to 1.000.

In addition, at two points of the spectrum intensity at 7110 eV and the spectrum intensity at 7111 eV at 150°C, the ratio of (spectrum intensity at 150°C) / (spectrum intensity at room temperature) is preferably 0.930 to 1.000, more preferably 0.950 to 1.000, and further preferably 0.970 to 1.000.

At the two points, when the ratios between the spectrum intensity at the temperature (100°C or 150°C) after the temperature increase and the spectrum intensity at room temperature are approximately the same, it can be said that the rise of the peak at the temperature after the temperature increase and the rise of the peak at room temperature are the same.

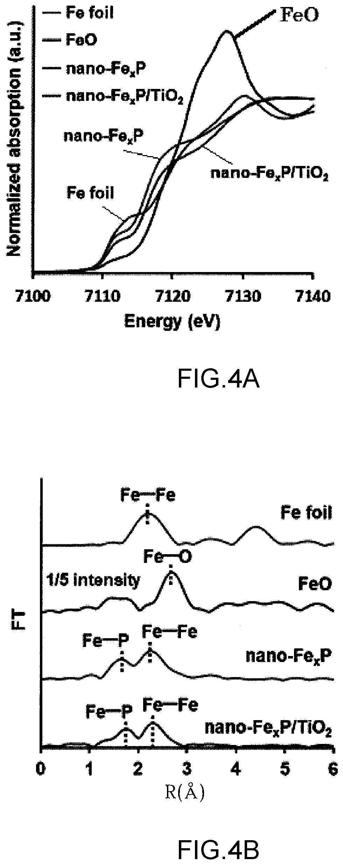

In the iron phosphide nanoparticles of the present invention, from the result of a spectrum of FT-EXAFS (Fourier transforms of extended X-ray absorption fine structure), the interatomic distance of Fe-Fe is considered to be about 2.65 Å as shown in FIG. 4B.

The iron phosphide nanoparticles of the present invention can, after having been used as a reduction catalyst (e.g., hydrogenation catalyst), maintain activity after the use, and can be recovered. Therefore, the iron phosphide nanoparticles can be recovered again to be reused. The recovery method is not particularly limited, and a known method such as filtration can be used. In addition, the iron phosphide nanoparticles of the present invention have high catalytic activity even after being recovered and reused, and thus are excellent in durability.

Another embodiment of the present invention relates to a composite body containing any of the iron phosphide nanoparticles described above and a carrier.

Since the iron phosphide nanoparticles of the present invention are excellent in adsorption ability, the type of the carrier is not limited, and the iron phosphide nanoparticles, when used in a reduction reaction as the composite body, exhibit effects as a reduction catalyst. The reason for excellence in adsorption ability is not clear, but that the iron phosphide nanoparticles are nano-sized and are stable under an atmospheric condition is also considered to be a part of the reason.

In the composite body of the present invention, iron atoms contained in the iron phosphide nanoparticles are in a low valence state, and the iron phosphide nanoparticles are stable under an atmospheric condition. Therefore, unlike the conventional technology, the iron phosphide nanoparticles of the present invention do not have circumstances in which: iron particles having catalytic activity are synthesized on a carrier and, immediately after the synthesis, have to be instantaneously used as a catalyst; or, in order to use the iron catalyst in a reduction reaction, pre-reduction treatment under high temperature and high pressure is essential.

Therefore, different from the conventional technology in which the type of the carrier is limited due to the use condition of iron particles as an iron catalyst, in the composite body of the present invention, when the composite body is used as a catalyst, the type of the carrier to be used in combination with the iron phosphide nanoparticles is not limited. This is one of the advantageous effects of the present invention. Thus, since there are no special circumstances, i.e., the reason why the carrier has to be limited is eliminated, and the catalytic activity cannot be obtained unless the carrier is limited, the type of the carrier is not limited and many types of carriers can be used in the composite body of the present invention.

In the composite body of the present invention, the type of the carrier is not limited as long as the carrier can be used as a support body that supports the iron phosphide nanoparticles. As the carrier, a carrier that is a liquid or a solid at normal temperature is preferable, and a carrier that is a solid is more preferable. In another embodiment, an example of the carrier is a carrier that does not have catalytic activity by itself, when used alone. When the iron phosphide nanoparticles are used together with the carrier, aggregation of the iron phosphide nanoparticles can be effectively inhibited, recovery as the composite body becomes easier, and reusability can be more enhanced.

An example of the carrier is at least one type selected from the group consisting of a polymer, a chalcogen compound, a metal compound, a metal, and a solid carbon material. As the carrier, one type may be used alone, or two types or more may be used in combination. As the carrier, a commercial product may be used.

The polymer may be either of a natural polymer and a synthetic polymer.

Examples of the natural polymer include organic natural polymers such as natural rubber, protein, starch, and cellulose; and inorganic natural polymers such as quartz, mica, and feldspar.

Examples of the synthetic polymer include: organic synthetic polymers such as polyacrylic acid, polymethacrylic acid, polyvinyl alcohol, polyvinyl chloride, polyvinyl pyrrolidone, polyethylene glycol (PEG), polypropylene glycol (PPG), polyethylene terephthalate (PET), polyester, polyethylene, polypropylene, polystyrene, polyurethane, polyethyleneimine, polyether sulfone, polyphenylene sulfide (PPS), polyether ether ketone (PEEK), polyvinylidene fluoride, polylactic acid, epoxy resin, fluorine-based resin (PTFE (polytetrafluoroethylene), PFA, FEP, PCTFE, ETFE, ECTFE, etc.), nylon resin, polyamide, polyimide, and rubber (silicone rubber, nitrile rubber, butyl rubber, butadiene rubber, etc.); inorganic synthetic polymers such as glass and silica gel; and the like.

The synthetic polymer may be a homopolymer having one type of monomer as the constitutional unit, or may be a copolymer having two types or more of monomers as the constitutional unit.

The polymer may be a thermoplastic resin or may be a thermosetting resin.

The weight-average molecular weight (Mw) of the polymer is not particularly limited, and an oligomer or a polymer having a weight-average molecular weight of less than 1000 to 10000, or less than 10000 to 1000000, may be adopted, or an ultrahigh molecular weight polymer having a weight-average molecular weight of 1000000 to 7000000 may be adopted.

The weight-average molecular weight means the weight-average molecular weight in terms of polystyrene obtained through gel permeation chromatography (GPC).

The chalcogen compound means a compound that contains a group 16 element (oxygen, sulfur, selenium, tellurium, polonium, livermorium). The group 16 element contained in the chalcogen compound may be of one type or may be of two types or more. The chalcogen compound is not particularly limited, and examples thereof include a compound having a structure of H₂X¹O₄ (X¹= a chalcogen excluding tellurium), H₆TeO₆, H₂X²O₃ (X²=a chalcogen) or salts thereof, a compound represented by MX₃ (in the formula, M is Ti, Zr, Hf, V, Nb, Ta, Mo, or W, and X is S or Se), a metal chalcogenide represented by MPX₃ (in the formula, M is Mg, V, Mn, Fe, Co, Ni, Zn, Cd, or In, and X is S or Se), and the like.

The metal compound is not particularly limited, and examples thereof include oxides of La, Ce, Pr, Nd, Pm, Sm, Eu, Gd, Tb, Dy, Ho, Er, Tm, Yb, Lu, Y, Si, Zr, Fe, Ti, Al, Mg, Co, Ni, Mn, Cr, Mo, W, V, Zn and Sn, solid solutions thereof, composite oxides thereof, and the like. Specific examples are SiO₂, TiO₂, Al₂O₃, and the like. In the present invention, semimetals, such as "Si", in the periodic table are included in metals.

Examples of the metal include Fe, metal alloys (stainless steel, etc.), and the like. The metal can be used as a metal-made carrier.

Examples of the solid carbon material include silicon carbide (SiC), activated carbon (active carbon), graphite, diamond, fullerene, carbon nanotube, graphene, amorphous carbon, and the like.

The shape and size of the carrier can be changed in accordance with a use form, and are not particularly limited. For example, the carrier may be a sheet or a film, or in a flat plate shape. The size of the carrier may be nano-sized, and may be 1 µm or more, 1 mm or more, or 1 cm or more. The carrier may be a nano-sheet (e.g., having a thickness of about 1 nm to 100 nm), for example.

In the composite body of the present invention, iron atoms contained therein are in a low valence state, and the composite body is stable under an atmospheric condition. The composite body has catalytic activity as a reduction catalyst.

The composite body of the present invention can be produced, without being particularly limited, in accordance with the type of the carrier. As a certain embodiment, a production method, for the composite body, in which the iron phosphide nanoparticles are left to stand or fixed on the carrier, is exemplified. The method of fixing the iron phosphide nanoparticles on the carrier is not particularly limited, and may be heat treatment or pressure-bonding treatment, for example. The heating temperature may be 100°C or less, for example.

As the carrier, a solid carrier (e.g., in a sheet shape) having a sufficiently larger size than the iron phosphide nanoparticles may be used, for example.

Since the iron phosphide nanoparticles of the present invention are excellent in adsorption ability, a known support method to a carrier can be used without particular limitation. In any production method for the composite body, no particular limitation is imposed, and for example, heat treatment, etc. may be performed. The heating temperature may be 150°C or less, or may be 120°C or less, for example.

The organic solvent to be used in the production method for the composite body is not particularly limited, and a polar organic solvent or a nonpolar organic solvent can be used. As the organic solvent, one type may be used alone, or two types or more may be used in combination.

Examples of the organic solvent to be used in the production method for the composite body include:
non-aromatic hydrocarbon solvents (examples: alkanes such as pentane, hexane, heptane, octane, nonane, decane, dodecane, isododecane, and tridecane, cycloalkanes such as cyclohexane and methylcyclohexane, decahydronaphthalene, and liquid paraffin);
aromatic hydrocarbon solvents (examples: benzene, toluene, xylene, diethylbenzene, mesitylene, tetralin, indene, naphthalene, and methylnaphthalene);
halogenated hydrocarbon solvents (examples: dichloromethane, dichloroethane, chloroform, and chlorobenzene);
alcohol solvents (examples: monohydric alcohols such as ethanol, propanol, 1-butanol, 2-butanol, 1-heptanol, 2-heptanol, 3-heptanol, 1-hexanol, 2-hexanol, benzyl alcohol, and oleyl alcohol; polyhydric alcohols such as ethylene glycol, polyethylene glycol, propylene glycol, polypropylene glycol, and glycerin);
ether solvents (examples: diethyl ether, diisopropyl ether, methyl t-butyl ether, diisoamyl ether, ethylene glycol derivatives (examples: monoglyme (ethyleneglycoldimethylether), methyl cellosolve, diethyl cellosolve, diglyme, diethylene glycol monoethyl ether, diethylene glycol diethyl ether, triglyme, tetraglyme, diethylene glycol monobutyl ether, triethylene glycol monomethyl ether, ethylene glycol monobenzyl ether, ethylene glycol monophenyl ether, diethylene glycol monobenzyl ether, and poly(ethylene glycol) monomethyl ether), propylene glycol derivatives (examples: 3-hexanol propylene glycol monopropyl ether, dipropylene glycol monoethyl ether, and tripropylene glycol monomethyl ether), 1,1-dimethoxy cyclohexane, phenetole, veratrole, dioxane, and tetrahydrofuran);
ester solvents (examples: ethyl acetate, butyl acetate, isopropyl acetate, 3-methoxy-3-methylbutyl acetate, dimethyl carbonate, diethyl malonate, ethylene carbonate, propylene carbonate, γ-butyrolactone, and α-acetyl-γ-butyrolactone);
ketone solvents (examples: acetone, methyl ethyl ketone, methyl isobutyl ketone, diisobutyl ketone, acetophenone, propiophenone, and isophorone);
sulfur-containing solvents (examples: dimethyl sulfoxide, sulfolane, and diphenyl sulfide); and
nitrogen-containing solvents (examples: amide solvents such as N,N-dimethylformamide, N,N-dimethylacetamide, N-methyl pyrrolidone, 1,3-dimethyl-2-imidazolidinone, N,N-dimethylacrylamide, N,N-dimethylacetoacetamide, N,N-diethylformamide, N,N-diethylacetamide, hexamethylphosphoramide, and methyl pyrrolidone; amine solvents such as triethanolamine; nitrile solvents such as acetonitrile and benzonitrile; nitro solvents such as nitrobenzene and o-nitrotoluene; and quinoline, tetrahydroquinoline, and dimethylimidazolidinone), and the like.

Another embodiment of the present invention relates to a production method for the iron phosphide nanoparticles.

An example of the production method for the iron phosphide nanoparticles of the present invention is a production method including: mixing a phosphorus compound and a surfactant together under heating; heating an obtained mixture; and further mixing an iron carbonyl compound thereto and heating a resultant matter, wherein 1-octadecene is not used.

Non Patent Literature 1 discloses a method for producing metal nanoparticles by using 1-octadecene as an essential component. However, the present inventors confirmed that iron nanoparticles obtained by the production method using 1-octadecene cannot be obtained in an amount that allows sufficient catalytic activity to be exhibited. Therefore, in the production method for the iron phosphide nanoparticles of the present invention, 1-octadecene is not used.

By not using 1-octadecene, the obtained iron phosphide nanoparticles have excellent catalytic activity and can be used as a reduction catalyst.

The production method for the iron phosphide nanoparticles of the present invention does not require another additive such as 1-octadecene.

In the production method for the iron phosphide nanoparticles of the present invention, as the phosphorus compound to be used as a phosphorization agent, a phosphorous acid ester compound is preferable.

Examples of the phosphorous acid ester compound include triphenyl phosphite, diphenyl mono(2-ethylhexyl)phosphite, diphenyl monodecyl phosphite, diphenyl mono(tridecyl)phosphite, tritolyl phosphite(tri-o-tolyl phosphite, tri-m-tolyl phosphite, tri-p-tolyl phosphite), trixylyl phosphite, tris(2,4-di-t-butylphenyl)phosphite, tris(2,6-dimethylphenyl)phosphite, tris(2-t-butylphenyl)phosphite, tris(2-t-butyl-5-methylphenyl)phosphite, trimethyl phosphite, triethyl phosphite, tripropyl phosphite, triisopropyl phosphite, and the like. Aryl phosphites such as triphenyl phosphite, diphenyl mono(2-ethylhexyl)phosphite, diphenyl monodecyl phosphite, diphenyl mono(tridecyl)phosphite, tritolyl phosphite, trixylyl phosphite, tris(2,4-di-t-butylphenyl)phosphite, tris(2,6-dimethylphenyl)phosphite, tris(2-t-butylphenyl)phosphite, and tris(2-t-butyl-5-methylphenyl)phosphite are preferable. Triaryl phosphites such as triphenyl phosphite, tritolyl phosphite, trixylyl phosphite, tris(2,4-di-t-butylphenyl)phosphite, tris(2,6-dimethylphenyl)phosphite, tris(2-t-butylphenyl)phosphite, and tris(2-t-butyl-5-methylphenyl)phosphite are more preferable. Triphenyl phosphite is further preferable. As the phosphorus compound, one type may be used alone, or two types or more may be used in combination.

As the surfactant, an alkylamine is used. The alkylamine is not particularly limited, but from the viewpoint of easy obtainment of rod-shaped particles as the iron phosphide nanoparticles, those having an alkyl group having 1 to 20 carbon atoms are preferable. From the viewpoint of more excellence in the catalytic activity of the iron phosphide nanoparticles, an alkyl group having 1 to 18 carbon atoms are more preferable, and an alkyl group having 1 to 16 carbon atoms are further preferable.

As a certain embodiment, a production method, for the iron phosphide nanoparticles, in which an alkylamine having an alkyl group having 1 to 14 carbon atoms is used as a surfactant is exemplified. As the alkylamine, one type may be used alone, or two types or more may be used in combination.

The alkyl group of the alkylamine may be linear, branched, or cyclic. Examples of the alkyl group include methyl group, ethyl group, n-propyl group, isopropyl group, n-butyl group, isobutyl group, sec-butyl group, tert-butyl group, n-pentyl group, isopentyl group, sec-pentyl group, neopentyl group, tert-pentyl group, 1-ethylpropyl group, 1,1-dimethylpropyl group, 1,2-dimethylpropyl group, n-hexyl group, 1-methylpentyl group, 2-methylpentyl group, 3-methylpentyl group, 4-methylpentyl group (isohexyl group), 1-ethylbutyl group, 2-ethylbutyl group, 1,1-dimethylbutyl group, 1,2-dimethylbutyl group, 1,3-dimethylbutyl group, 1,4-dimethylbutyl group, 2,2-dimethylbutyl group, 2,3-dimethylbutyl group, 3,3-dimethylbutyl group, 1-ethyl-2-methyl-propyl group, 1,1,2-trimethylpropyl group, n-heptyl group, 2-methylhexyl group, n-octyl group, isooctyl group, tert-octyl group, 2-ethylhexyl group, 3-methylheptyl group, n-nonyl group, isononyl group, 1-methyloctyl group, 2-ethylheptyl group, n-decyl group, 1-methylnonyl group, n-undecyl group, 1,1-dimethylnonyl group, n-dodecyl group, n-tridecyl group, n-tetradecyl group, n-pentadecyl group, n-hexadecyl group, n-heptadecyl group, n-octadecyl group, n-nonadecyl group, n-eicosyl group, n-cyclopentyl group, n-cyclopentylmethyl group, cyclohexyl group, cyclohexylmethyl group, and the like.

The alkyl group may have a substituent or may be unsubstituted. The number of substituents can be changed in accordance with the number of carbon atoms in the alkyl group, and may be 1 to 10, may be 1 to 5, or may be 1 to 3. Examples of the substituent include a halogen atom, a cyano group (nitrile group), a lower alkyl group, a halo lower alkyl group, a hydroxy lower alkyl group, a hydroxy group, a halo lower alkoxy group, and the like.

The step of mixing the phosphorus compound and the surfactant under heating is not particularly limited, and can be performed using a known method and a known apparatus. The mixing step may be performed under a vacuum condition.

The use amount of the surfactant may be 0.05 to 20 equivalents (mole equivalents), may be 0.1 to 15 equivalents, or may be 0.5 to 13 equivalents, with respect to the use amount of the phosphorus compound.

The phosphorus compound and the surfactant are mixed together under heating (hereinafter, also referred to as heating step [1]). The heating temperature in the heating step [1] is preferably 80 to 280°C, more preferably 90 to 250°C, and further preferably 100 to 220°C. The heating temperature may be 180°C or less as a gentler condition. The heating temperature can be changed as appropriate in accordance with the target degree of phosphorization of the iron phosphide nanoparticles.

Preferably, the heating step [1] is performed under agitation from the viewpoint of promoting the reaction and being able to more easily produce the iron phosphide nanoparticles of the present invention. As a certain preferable embodiment, preferably, the heating step [1] is performed under a vacuum condition or in an argon atmosphere from the viewpoint of being able to more easily produce the iron phosphide nanoparticles of the present invention.

The reaction time in the heating step [1] is not particularly limited and can be changed as appropriate in accordance with the target degree of phosphorization of the iron phosphide nanoparticles. The reaction time is preferably 10 to 120 minutes, more preferably 15 to 90 minutes, and further preferably 20 to 80 minutes, for example.

Following the heating step [1], the obtained mixture is further heated (hereinafter, also referred to as heating step [2]). Preferably, the heating step [2] is performed in an argon atmosphere.

The heating temperature in the heating step [2] is not particularly limited as long as the heating temperature is a temperature higher than that in the heating step [1], but for example, is preferably 90 to 300°C, more preferably 100 to 290°C, and further preferably 120 to 270°C. The heating temperature can be changed as appropriate in accordance with the target degree of phosphorization of the iron phosphide nanoparticles. The heating temperature in the heating step [2] may be a temperature higher by 5°C or more or may a temperature higher by 10°C or more than that in the heating step [1].

The reaction time in the heating step [2] is not particularly limited and can be changed as appropriate in accordance with the target degree of phosphorization of the iron phosphide nanoparticles. The reaction time is preferably 10 to 120 minutes, more preferably 15 to 90 minutes, and further preferably 20 to 80 minutes, for example.

Following the heating step [2], an iron carbonyl compound is mixed thereto and a resultant matter is further heated (hereinafter, also referred to as heating step [3]).

Examples of the iron carbonyl compound include Fe(CO)s, Fe₂(CO)₉, Fe₃(CO)₁₂, and the like, and Fe(CO)s is preferable. As the iron carbonyl compound, one type may be used alone, or two types or more may be used in combination.

In a certain embodiment, in the heating step [3], preferably, the iron carbonyl compound is mixed after the temperature has been increased to a temperature higher than that in the heating step [2]. The heating temperature in the heating step [3] is preferably 200 to 500°C, more preferably 220 to 450°C, and further preferably 250 to 420°C. The heating temperature can be changed as appropriate in accordance with the target degree of phosphorization of the iron phosphide nanoparticles. The heating temperature in the heating step [3] may be a temperature higher by 5°C or more or may be a temperature higher by 10°C or more than that in the heating step [2].

The reaction time in the heating step [3] is not particularly limited, and can be changed as appropriate in accordance with the target degree of phosphorization of the iron phosphide nanoparticles. The reaction time is preferably 5 minutes to 10 hours, more preferably 10 minutes to 8 hours, and further preferably 15 minutes to 6 hours, for example.

The temperature increase rate when increasing the temperature in the heating step [3] after mixing of the iron carbonyl compound is not particularly limited, and can be changed as appropriate in accordance with the target degree of phosphorization of the iron phosphide nanoparticles. For example, in order to gently advance the reaction, the temperature increase rate can be set to be low. The temperature increase rate is preferably 5 to 150°C/minute, more preferably 10 to 100°C/minute, and further preferably 15 to 90°C/minute.

Preferably, the production method for the iron phosphide nanoparticles of the present invention further includes a washing step. As the washing liquid to be used in the washing step, an organic solvent can be used. The organic solvent is not particularly limited, and examples thereof include the same organic solvents as those usable in the production method for the composite body described above. As the organic solvent, one type may be used alone, or two types or more may be used in combination. When two types or more are used in combination, the mixing ratio is not particularly limited, but chloroform and acetone can be used at a volume ratio of 1:1, for example. An example of the washing step is a method in which the product obtained after the heating step [3] is centrifuged by using the organic solvent.

After the washing step, the product may be subjected to vacuum drying as necessary, to obtain powdery iron phosphide nanoparticles.

Another embodiment of the present invention relates to a reduction catalyst that contains any of the iron phosphide nanoparticles described above. Examples of the reduction reaction using the reduction catalyst include a reduction reaction of nitrogen oxide (NOx), a hydrogenation reaction, and the like.

In a certain embodiment, the reduction catalyst of the present invention may contain only the iron phosphide nanoparticles. As the reduction catalyst, the iron phosphide nanoparticles may be used as they are. In another embodiment, the reduction catalyst of the present invention may contain the iron phosphide nanoparticles and a carrier, and the iron phosphide nanoparticles and the carrier may form a composite body.

An example in which the reduction catalyst of the present invention is used as a hydrogenation catalyst will be described below.

In the present description, unless particularly mentioned, "reduction catalyst" can be read as "hydrogenation catalyst". As a certain embodiment, a hydrogenation catalyst that contains any of the iron phosphide nanoparticles described above is exemplified. As another embodiment, a hydrogenation catalyst that contains any of the iron phosphide nanoparticles described above and a carrier, wherein the iron phosphide nanoparticles and the carrier form a composite body, is exemplified.

The reduction catalyst is not limited to the hydrogenation catalyst.

As a certain embodiment, a production method, for a hydrogenated organic compound, in which an organic compound is hydrogenated by using the hydrogenation catalyst described above, to obtain the hydrogenated organic compound, is exemplified.

Examples of the organic compound include a nitrile compound, an aldehyde compound, an unsaturated compound, and the like.

Examples of the hydrogenated organic compound produced through the hydrogenation include a primary amine compound, an alcohol compound, a saturated compound, and the like.

Specific examples of the production method for the hydrogenated organic compound include: a production method, for a primary amine compound, in which a nitrile compound is hydrogenated by using the hydrogenation catalyst described above to obtain the primary amine compound; a production method, for an alcohol compound, in which an aldehyde compound is hydrogenated by using the hydrogenation catalyst described above to obtain the alcohol compound; and a production method, for a saturated compound, in which an unsaturated compound is hydrogenated by using the hydrogenation catalyst described above to obtain the saturated compound.

Using an example in which the organic compound is a nitrile compound, the production method, for a primary amine compound, in which the nitrile compound is hydrogenated by using the hydrogenation catalyst (the iron phosphide nanoparticles or the composite body) described above to obtain the primary amine compound will be described below.

Examples of the nitrile compound include a nitrile compound (mononitrile compound) having one cyano group, a nitrile compound (dinitrile compound) having two cyano groups, a nitrile compound (trinitrile compound) having three cyano groups, a nitrile compound having four or more cyano groups, and the like.

Examples of the nitrile compound having one cyano group include an aliphatic nitrile compound, an aromatic nitrile compound having one or two aromatic rings and/or heterocyclic rings optionally having a substituent, and the like. In the present description, a nitrile compound that has an aromatic ring or a heterocyclic ring is encompassed by the aromatic nitrile compound.

Examples of the aliphatic nitrile compound include an aliphatic nitrile compound having 1 to 30 carbon atoms excluding the cyano group. The aliphatic nitrile compound having 1 to 30 carbon atoms excluding the cyano group may be linear, branched, or cyclic. The aliphatic nitrile compound having 1 to 30 carbon atoms excluding the cyano group may be substituted by a halogen atom or may be unsubstituted. The number of carbon atoms excluding the cyano group of the aliphatic nitrile compound is not particularly limited, and may be 1 to 20 or may be 1 to 15.

Examples of the aliphatic nitrile compound as the mononitrile compound include linear or branched aliphatic nitriles such as acetonitrile, propionitrile, butyronitrile, isobutyronitrile, pentanenitrile (valeronitrile), isovaleronitrile, hexanenitrile, heptanenitrile, octanenitrile, decanenitrile, lauronitrile, octadecanenitrile, 3-methoxypropionitrile, 2-methylbutyronitrile, trimethylacetonitrile, cyclopentanecarbonitrile, cyclohexanecarbonitrile, fluoroacetonitrile, difluoroacetonitrile, trifluoroacetonitrile, 2-fluoropropionitrile, 3-fluoropropionitrile, 2,2-difluoropropionitrile, 2,3-difluoropropionitrile, 3,3-difluoropropionitrile, 2,2,3-trifluoropropionitrile, 3,3,3-trifluoropropionitrile, 3,3'-oxydipropionitrile, 3,3'-thiodipropionitrile, pentafluoropropionitrile, and methoxyacetonitrile; and cyclic aliphatic nitriles such as cyclopentanecarbonitrile, cyclohexanecarbonitrile, and 1-adamantylcarbonitrile.

Examples of the aromatic ring of the aromatic nitrile compound having one or two aromatic rings optionally having a substituent as the mononitrile compound include benzene, naphthalene, biphenyl, and the like. Examples of the heterocyclic ring of the aromatic nitrile compound having one or two heterocyclic rings optionally having a substituent include: heterocyclic rings including only nitrogen atoms as the heteroatom such as five-membered rings such as pyrrole, imidazole, and pyrazole, and six-membered rings such as pyridine, pyridazine, pyrimidine, and pyrazine; a heterocyclic ring including only an oxygen atom as the heteroatom such as furan; a heterocyclic ring including only a sulfur atom as the heteroatom such as thiophene; a heterocyclic ring including a nitrogen atom and an oxygen atom as the heteroatom such as oxazole; heterocyclic rings including a nitrogen atom and a sulfur atom as the heteroatom such as thiazole and isothiazole; condensed heterocyclic rings such as benzothiazole, benzoxazole, benzimidazole, quinoline, quinoxaline, chroman, and indole; and the like.

Examples of the substituent include an alkyl group having 1 to 6 carbon atoms, a phenyl group, a phenylalkyl group having 7 to 10 carbon atoms, an alkoxy group having 1 to 6 carbon atoms, a halogen atom, a hydroxy group, an amino group, a substituent amino group of an alkyl group having 1 to 6 carbon atoms, a formyl group, an acyl group, a carboxyl group, a cyano group, a nitro group, a sulfo group, an alkylthio group of an alkyl group having 1 to 6 carbon atoms, an azo group, an azido group, and the like. The number of substituents can be selected as appropriate in accordance with the structure of the compound, and may be 1 to 6, may be 1 to 4, or may be 1 to 3, for example.

Examples of the aromatic nitrile compound having one or two aromatic rings and/or heterocyclic rings optionally having a substituent include benzonitrile; alkylbenzonitriles such as p-trinitrile, methylbenzonitrile(2-methylbenzonitrile, 3-methylbenzonitrile, 4-methylbenzonitrile), 4-ethylbenzonitrile, 4-t-butylbenzonitrile, and 4-(aminomethyl)benzonitrile; alkoxybenzonitriles such as methoxybenzonitrile (o-methoxybenzonitrile, m-methoxybenzonitrile, p-methoxybenzonitrile); mercaptobenzonitriles such as mercaptobenzonitrile and 2,5-dimercaptobenzonitrile; alkylthiobenzonitriles such as 4-(methylthio)benzonitrile; halogenated benzonitriles such as bromobenzonitrile (o-bromobenzonitrile, m-bromobenzonitrile, p-bromobenzonitrile), chlorobenzonitrile (o-chlorobenzonitrile , m-chlorobenzonitrile, p-chlorobenzonitrile), dichlorobenzonitrile (2,3-dichlorobenzonitrile, 2,4-dichlorobenzonitrile, 2,5-dichlorobenzonitrile, 2,6-dichlorobenzonitrile, 3,4-dichlorobenzonitrile, 3,5-dichlorobenzonitrile), trichlorobenzonitrile (2,3,4-trichlorobenzonitrile, 2,3,5-trichlorobenzonitrile, 2,3,6-trichlorobenzonitrile, 2,4,6-trichlorobenzonitrile), fluorobenzonitrile (o-fluorobenzonitrile, m-fluorobenzonitrile, p-fluorobenzonitrile), difluorobenzonitrile (2,3-difluorobenzonitrile, 2,4-difluorobenzonitrile, 2,5-difluorobenzonitrile, 2,6-difluorobenzonitrile, 3,4-difluorobenzonitrile, 3,5-difluorobenzonitrile), trifluorobenzonitrile (2,3,4-trifluorobenzonitrile, 2,3,5-trifluorobenzonitrile, 2,3,6-trifluorobenzonitrile, 2,4,6-trifluorobenzonitrile), trifluoromethylbenzonitrile, and 4-(3-chloro-4-methylphenyl)benzonitrile; acetoxybenzonitrile, phenylacetonitrile, 2-phenylbutyronitrile, 3-phenylpropionitrile, 4-phenylbenzonitrile, phenoxybenzonitrile (2-phenoxybenzonitrile, 3-phenoxybenzonitrile, 4-phenoxybenzonitrile), 4-(4-methylphenyl)benzonitrile, 2-(p-tolyl)benzonitrile, 2-naphthalenecarbonitrile, 6-methoxy-2-naphthalenecarbonitrile, 2-pyridinecarbonitrile, 3-pyridinecarbonitrile, 4-pyridinecarbonitrile, 6-chloro-2-pyridinecarbonitrile, 6-methoxy-3-pyridinecarbonitrile, pyrrole-2-carbonitrile, 1H-indole-3-carbonitrile, 1H-indole-6-carbonitrile, piperonylonitrile, 2-furancarbonitrile, 3-furancarbonitrile, 2-thiophenecarbonitrile, 3-thiophenecarbonitrile, 3-methylthiophene-2-carbonitrile, thiophene-2-acetonitrile, and thiophene-3-acetonitrile; and the like.

Examples of the aliphatic nitrile compound as the dinitrile compound include linear or branched alkyl dinitriles having 1 to 30 carbon atoms excluding the cyano group, such as malononitrile, pentanedinitrile, hexanedinitrile (adiponitrile), heptanedinitrile, octanedinitrile, and decanedinitrile; cycloalkyl dinitriles having 3 to 30 carbon atoms excluding the cyano group such as 1,4-cyclohexane-dicarbonitrile; and the like. The number of carbon atoms excluding the cyano group of the aliphatic nitrile compound is not particularly limited, and may be 1 to 20 or may be 1 to 15.

Examples of the aromatic nitrile compound having one or two aromatic rings and/or heterocyclic rings optionally having a substituent as the dinitrile compound include: aromatic nitrile compounds having one aromatic ring and/or heterocyclic ring such as 1,2-benzene dicarbonitrile, 1,3-benzene dicarbonitrile, 1,4-benzene dicarbonitrile, and 2,5-difluoro-1,4-benzene dicarbonitrile; aromatic nitrile compounds having two aromatic rings and/or heterocyclic rings such as 2-benzhydrylcyclohexane-1,1-dicarbonitrile; and the like.

From the viewpoint of having sufficient catalytic activity, the use amount of the hydrogenation catalyst, with respect to 100 mol% of the nitrile compound, is preferably 0.1 to 15 mol%, more preferably 0.15 to 12 mol%, and further preferably 0.2 to 10 mol%, in terms of the amount of Fe. In the above range, the hydrogenation reaction sufficiently proceeds. The hydrogenation catalyst of the present invention functions as a catalyst even in a very small amount, and is excellent in catalytic activity.

Preferably, the production method for the primary amine compound is performed through heating. The heating temperature is not particularly limited, but preferably 80 to 350°C, more preferably 90 to 280°C, and further preferably 100 to 250°C. When the reaction is carried out under a more gentle condition, 200°C or less may be adopted.

The reaction time is not particularly limited, and may be about 5 minutes to 60 hours, may be about 10 minutes to 30 hours, or may be about 20 minutes to 15 hours. In accordance with the purpose, the reaction time may be set to 8 hours or less.

In the production method for the primary amine compound, the hydrogen pressure is 8 MPa or less, and when the production method is performed under a more gentle condition, the hydrogen pressure may be 5 MPa or less, may be 4.5 MPa or less, may be 3.0 MPa or less, or may be 1.0 MPa or less.

The pressure during the reaction is not particularly limited, but, for example, preferably, H₂:NH₃ is 0.5 MPa:0.01 MPa to 7 MPa:0.9 MPa, more preferably 0.8 MPa:0.02 MPa to 6 MPa:0.8 MPa, and further preferably 0.8 MPa:0.03 MPa to 5 MPa:0.7 MPa. In the above range, the hydrogenation reaction sufficiently proceeds.

As a certain preferable embodiment, a production method, for the hydrogenated organic compound, in which the nitrile compound is hydrogenated in a hydrogen atmosphere and in the presence of ammonia, at a hydrogen pressure of 8 MPa or less, is exemplified.

In the production method of the present invention, since the nitrile compound can also serve as a solvent, an additional solvent need not necessarily be used. In accordance with the type of a substrate, etc., the presence or absence and the type of a solvent can be selected.

As the solvent, for example, those shown as examples of the organic solvent to be used in the production method for the composite body can be used. Alcohol solvents such as methanol, ethanol, n-propanol, 2-propanol, n-butanol, 2-butanol, and t-butanol; ketone solvents such as acetone and methyl ethyl ketone; amide solvents such as N,N-dimethylformamide and N,N-dimethylacetamide; and ether solvents such as 1,2-dimethoxy ethane and diethylene glycol dimethyl ether are preferable, and an alcohol solvent is more preferable.

In the production method for the primary amine compound, the hydrogenation reaction may be performed in the presence of a base.

Examples of the base include organic bases ((a) tertiary amine, (b) nitrogen-containing aromatic heterocyclic compound, (c) compound having an imine skeleton (-C=N-C-), (in the present description, this compound will also be referred to as "imine-based base")), (d) inorganic base, (e) tetraalkylammonium hydroxide, and the like, but the base is not limited thereto. As the base, one type may be used alone, or two types or more may be used in combination.

Examples of (a) tertiary amine include trimethylamine, triethyl amine, N-ethyldiisopropylamine, tri-n-propylamine, triisopropylamine, tri-n-butylamine, trioctylamine, tridecylamine, triphenylamine, tribenzylamine, tris(2-ethylhexyl)amine, N,N-diisopropylethylamine, N,N-dimethyldecylamine, N-benzyldimethylamine, N-butyldimethylamine, N,N-dimethylcyclohexylamine, N,N,N',N'-tetramethylethylenediamine, N,N-dimethylaniline, N,N-diethylaniline, 1,4-diazabicyclo[2.2.2]octane, N-methylpyrrolidine, N-methylpiperidine, N-methylmorpholine, N-ethylmorpholine, N,N'-dimethylpiperazine, N-methyl pyrrolidone, N-vinyl-pyrrolidone, bis(2-dimethylamino-ethyl)ether, N,N,N,N',N"-pentamethyl diethylene triamine, triethanolamine, tripropanolamine, dimethylethanolamine, dimethylaminoethoxyethanol, N,N-dimethylaminopropylamine, N,N,N',N',N"-pentamethyldipropylenetriamine, tris(3-dimethylaminopropyl)amine, tetramethylimino-bis(propylamine), N-diethyl-ethanolamine, and the like.

Examples of (b) nitrogen-containing aromatic heterocyclic compound include pyridine, 2,4,6-trimethylpyridine, 4-dimethylaminopyridine, lutidine, pyrimidine, pyridazine, pyrazine, oxazole, isoxazole, thiazole, isothiazole, imidazole, 1,2-dimethylimidazole, 3-(dimethylamino)propylimidazole, pyrazole, furazan, quinoline, isoquinoline, purine, 1H-indazole, quinazoline, cinnoline, quinoxaline, phthalazine, pteridine, phenanthridine, 2,6-di-t-butylpyridine, 2,2'-bipyridine, 4,4'-dimethyl-2,2'-bipyridyl, 5,5'-dimethyl-2,2'-bipyridyl, 6,6'-t-butyl-2,2'-dipyridyl, 4,4'-diphenyl-2,2'-bipyridyl, 1,10-phenanthroline, 2,7-dimethyl-1,10-phenanthroline, 5,6-dimethyl-1,10-phenanthroline, 4,7-diphenyl-1,10-phenanthroline, and the like.

Examples of (c) imine-based base include 1,8-diazabicyclo[5.4.0]undeca-7-ene (diazabicycloundecene), 1,5-diazabicyclo[4.3.0]non-5-ene, and the like.

Examples of (d) inorganic base include: hydrides (sodium hydride, potassium hydride, lithium hydride, calcium hydride, etc.) of alkali metals or alkaline earth metals; hydroxides (sodium hydroxide, potassium hydroxide, lithium hydroxide, calcium hydroxide, etc.) of alkali metals or alkaline earth metals; carbonates (sodium carbonate, potassium carbonate, lithium carbonate, calcium carbonate, etc.) of alkali metals or alkaline earth metals; hydrogencarbonates (sodium hydrogencarbonate, potassium hydrogencarbonate, lithium hydrogencarbonate, etc.) of alkali metals; oxides (lithium oxide, sodium oxide, potassium oxide, calcium oxide, magnesium oxide) of alkali metals or alkaline earth metals; halides (lithium fluoride, sodium fluoride, potassium fluoride, cesium fluoride, magnesium fluoride, calcium fluoride, cesium chloride, etc.) of alkali metals or alkaline earth metals; alkoxides (lithium methoxide, sodium methoxide, potassium methoxide, lithium ethoxide, sodium ethoxide, potassium ethoxide, lithium isopropoxide, sodium isopropoxide, potassium isopropoxide, lithium tert-butoxide, sodium tert-butoxide, and potassium tert-butoxide) of alkali metals; and the like.

Examples of (e) tetraalkylammonium hydroxide include tetramethylammonium hydroxide, tetraethylammonium hydroxide, tetra n-propylammonium hydroxide, tetra n-butylammonium hydroxide, and the like.

With respect to 1 mol of the nitrile compound, the use amount of the base is preferably 0.01 to 10 mol, more preferably 0.02 to 8 mol, and further preferably 0.05 to 4 mol. In the above range, the hydrogenation reaction sufficiently proceeds.

As long as the effects of the present invention are exhibited, the present invention encompasses embodiments in various combinations of the above configurations within the scope of the technical concept of the present invention.

### [Example]

Next, the present invention will be further specifically described by means of Examples, but the present invention is not limited to these Examples, and many modifications can be made within the technical concept of the present invention by those having ordinary knowledge in the art.

### [Example 1: iron phosphide nanoparticles]

### <Example 1-1>

Iron phosphide nanoparticles were produced by the following method. First, 10 equivalents (mole equivalent) of triphenyl phosphite and hexadecyl amine were put in a Schlenk tube and agitated at 120°C for 30 minutes under a vacuum condition. Next, the temperature was increased to 200°C in an argon atmosphere and then Fe(CO)s was added. Further, the temperature was increased to 320°C at 50°C/minute, and reaction was carried out for 4 hours, to obtain a mixed liquid.

The mixed liquid was cooled to room temperature, and the product was allowed to precipitate in acetone, whereby iron phosphide nanoparticles were isolated. The obtained iron phosphide nanoparticles (hereinafter, also referred to as "nano-FeₓP") were evaluated by the following method.

### <Electron microscopic observation of iron phosphide nanoparticles>

With respect to the iron phosphide nanoparticles obtained in the Example, observation was performed using a field emission transmission electron microscope (trade name "Tecnai (registered trademark) G2 20ST", acceleration voltage: 200 kV, manufactured by FEI Company (now: Thermo Fisher Scientific (US)). The result is shown in FIG. 1(a).

In addition, with respect to the iron phosphide nanoparticles obtained in the Example, observation by a high-resolution transmission electron microscopy (HRTEM) was also performed. The result is shown in FIG. 1 (c) together with a result of a selected area electron diffraction (SAED) pattern. From FIG. 1(c), it was confirmed that a single crystal of iron phosphide having a hexagonal structure was obtained.

Further, with respect to the iron phosphide nanoparticles obtained in the Example, observation by high-angle annular dark field scanning TEM (HAADF-STEM) was also performed. The result is shown in FIG. 1(d). From FIG. 1(d), as in the TEM image in FIG. 1(a), it was confirmed that nanoparticles were formed.

### <Elementary analysis of iron phosphide nanoparticles>

With respect to the iron phosphide nanoparticles obtained in the Example, using an ultrasensitive EDX system, a scanning transmission electron microscope (STEM) image including an element map was obtained. Specifically, element mapping was performed using a transmission electron microscope (single atom analysis transmission electron microscope, trade name "Titan Cubed G2 60-300", acceleration voltage: 300 kV, manufactured by FEI Company (now: Thermo Fisher Scientific (US)) including a super-X energy-dispersive X-ray spectroscopy (EDX) detector. The result is shown in FIGS. 1(e) and 1(f). FIG. 1(e) shows an analysis result of iron atoms. FIG. 1(f) shows an analysis result of phosphorus atoms. From FIGS. 1(e) and 1(f) which are the results of visualization of the respective distributions of iron atoms and phosphorus atoms in the iron phosphide nanoparticles, it was confirmed that iron atoms and phosphorus atoms were uniformly present in the iron phosphide nanoparticles.

### <Quantitative analysis of iron phosphide nanoparticles>

With respect to the TEM image, an energy-dispersive X-ray analysis (EDX) was performed using "Quantax" (software: ESPRIT Spectrum) manufactured by BRUKER.

### [Measurement condition]

acceleration voltage: 300 kV

### <Powder X-ray diffraction measurement of iron phosphide nanoparticles>

With respect to the iron phosphide nanoparticles obtained in the Example, about 40 mg of a sample was placed on a glass sample plate, and an X-ray diffraction measurement was performed, without performing pretreatment, under the following condition, using a fully automatic multipurpose X-ray diffraction apparatus (trade name "Philips X'PERT MPD diffractometer" manufactured by PHILIPS KK.) under an atmospheric condition, and using CuKα radiation (λ: 1.5405 Å). The result is shown in FIG. 2A.

### [Measurement condition]

tube voltage: 45 kV
tube current: 40 mA
measurement temperature: room temperature
range of measurement angle: 20.00 to 70.00°
sampling interval: 0.013°
scanning speed: 0.42°/minute

As shown in FIG. 2A and FIG. 2B, with respect to the iron phosphide nanoparticles obtained in the Example, in a powder X-ray diffraction measurement using CuKα radiation under a condition of presence of oxygen, the intensity at the peak (diffraction angle (2θ): 40.2°) corresponding to the (111) plane of Fe₂P was observed as the maximum intensity.

In addition, characteristic peaks (diffraction angle (2θ): 48.3° and 32.7°) corresponding to the (211) plane and the (011) plane of FeP were observed.

### <X-ray photoelectron spectroscopy (XPS) of iron phosphide nanoparticles>

With respect to the iron phosphide nanoparticles obtained in the Example, a measurement according to XPS was performed using an X-ray photoelectron analyzer (trade name "KRATOS ULTRA2", SHIMADZU CORPORATION) and using AlKα radiation as an excitation source. The result is shown in FIG. 3.

As shown in FIG. 3, it was confirmed that, in the Fe2p_{3/2} spectrum, contained iron atoms had a peak at the position of 706.8 eV. From the result of the XPS, a low valence state was confirmed to be present at the surface (depth: up to about 10 nm) of the iron phosphide nanoparticles.

### [Example 2: composite body]

### <Example 2-1: composite body of iron phosphide nanoparticles and TiO₂>

A composite body was produced using the iron phosphide nanoparticles produced in Example 1-1 and TiO₂ as a carrier. Specifically, the powder of the iron phosphide nanoparticles produced in Example 1-1 was dissolved in n-hexane. TiO₂ having an average particle diameter in a range of 0.01 µm to 1 µm was added thereto, such that the mass ratio of iron phosphide nanoparticles:TiO₂=1:25 was realized. Then, the resultant matter was agitated at 25°C for 6 hours, to obtain a composite body of the iron phosphide nanoparticles and TiO₂.

The average particle diameter of TiO₂ can be calculated by a laser diffraction scattering method. Specifically, for example, the average particle diameter of TiO₂ can be measured on a volume basis, using a 0.2% sodium hexametaphosphate aqueous solution as a dispersion medium, by a laser diffraction-type particle size distribution measurement apparatus (SALD-2300: manufactured by SHIMADZU CORPORATION).

With respect to the obtained composite body, observation was performed by the same method as in the TEM observation of the iron phosphide nanoparticles in Example 1-1. The result is shown in FIG. 1(b). From the image in FIG. 1(b), it was confirmed that 10 or more iron phosphide nanoparticles were present on TiO₂ serving as the carrier, thereby forming a composite body.

It was confirmed that the iron phosphide nanoparticles are excellent in atmospheric stability also from the fact that a composite body of the iron phosphide nanoparticles and TiO₂ was able to be produced at 25°C.

### <XANES measurement regarding K absorption edge of Fe atom in iron phosphide nanoparticles and composite body>

With respect to the iron phosphide nanoparticles obtained in the Example and the composite body obtained in Example 2-1, a XANES measurement regarding the K absorption edge of Fe atoms was performed under the following condition at the large synchrotron radiation facility "SPring-8" (beam line BL01B1, BL14B2, zip code 679-5198, 1-1, Kouto, Sayo-cho, Sayo-gun, Hyogo Prefecture). The result is shown in FIG. 4A. As a control, iron foil (Fe foil) was used. As a reference example, FeO was used.

### [Measurement condition]

The measurement was performed at room temperature, using an Si (111) monochromator of SPring-8 according to a transmission method.

### Measurement time:

Fe foil and FeO = 120 sec
nano-FeₓP and nano-FeₓP/TiO₂ = 600 sec

As shown in FIG. 4A, the rises of the peaks of the iron phosphide nanoparticles and the composite body were the same as the rise of the peak of the iron foil (Fe foil). In the result of the XANES measurement, as compared with the surface evaluated by the XPS, a deeper part can be evaluated. Therefore, from the result in FIG. 4A, it was confirmed that the iron atoms, i.e., not only the iron atoms present at the surface but also the iron atoms present on a more inner side, contained in the iron phosphide nanoparticles and the composite body were present in a low valence state.

### <FT-EXAFS measurement regarding K absorption edge of Fe atoms of iron phosphide nanoparticles and composite body>

An FT-EXAFS measurement was performed with respect to the iron phosphide nanoparticles obtained in Example 1-1 and the composite body obtained in Example 2-1.

### [Measurement condition]

The measurement was performed at room temperature, using the Si (111) monochromator of SPring-8 according to a transmission method.

### Measurement time:

Fe foil and FeO = 120 sec
nano-FeₓP and nano-FeₓP/TiO₂ = 600 sec

Through Fourier transform of the EXAFS vibration, a spectrum indicating a peak according to the interatomic distance was obtained. The result is shown in FIG. 4B. From the obtained result, the interatomic distance of Fe-P was 2.26 Å and the interatomic distance of Fe-Fe was 2.65 Å.

### <Example 2-2: composite body of iron phosphide nanoparticles and SiO₂>

A composite body of the iron phosphide nanoparticles and SiO₂ was obtained by the same method as in Example 2-1 except that SiO₂ having an average particle diameter in a range of 0.01 µm to 1 µm was used instead of TiO₂, such that the mass ratio of iron phosphide nanoparticles:SiO₂=1:25 was realized. The measurement method of the average particle diameter of SiO₂ was the same as the measurement method of the average particle diameter of TiO₂.

### <Example 2-3: composite body of iron phosphide nanoparticles and carbon>

A composite body of the iron phosphide nanoparticles and carbon was obtained by the same method as in Example 2-1 except that carbon (trade name "Activated Carbon (Powder)", manufactured by FUJIFILM Wako Pure Chemical Corporation) was used instead of TiO₂.

### [Example 3: production of benzylamine]

### <Example 3-1>

Benzonitrile was hydrogenated using the iron phosphide nanoparticles of Example 1-1 as a catalyst, to produce benzylamine. Specifically, 0.1 g of the iron phosphide nanoparticles (the amount of Fe was 0.036 mmol (the amount of Fe was 7.2 mol% with respect to 100 mol% of benzonitrile)) of Example 1-1, 3 ml of 2-propanol, and 0.5 mmol of benzonitrile were put in an autoclave, with hydrogen gas and NH₃ gas pressurized so as to realize H₂:NH₃=3.8 MPa:0.2 MPa, and heated to 180°C, and reaction was carried out for 2 hours. The result is shown in Table 1 below.

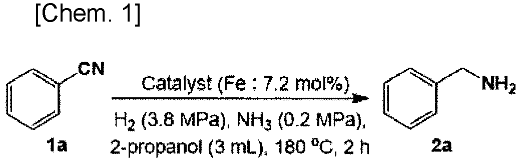

### <Examples 3-2 to 3-12>

Benzylamine was produced by the same method as in Example 3-1 except that the type of the catalyst that was used was changed to those produced in Examples 2-1 to 2-3 and that the presence or absence of addition (use amount: 0.1 mmol) of various bases was changed as shown in Table 1 below. As the composite bodies of Examples 3-2 to 3-4 and 3-7 to 3-12, the composite body obtained in Example 2-1 was used. The result is shown in Table 1 below.

### <Comparative Examples 3-1 to 3-3>

Benzylamine was produced by the same method as in Example 3-1 except that the type of the catalyst that was used was changed to those shown in Table 1 below. The result is shown in Table 1 below.

**[Table 1]**

| | Catalyst | Base | Conversion ratio (%)¹⁾ | Yield (%)¹⁾ |
|---|---|---|---|---|
| Example 3-1 | nano-FeₓP | - | 36 | 20 |
| Example 3-2 | nano-FeₓP/TiO₂ | - | 89 | 78 |
| Example 3-3 ²⁾ | nano-FeₓP/TiO₂ | - | >99 | 94 |
| Example 3-4 ³⁾ | nano-FeₓP/TiO₂ | - | >99 | 84 |
| Example 3-5 | nano-FeₓP/C | - | 45 | 28 |
| Example 3-6 | nano-FeₓP/SiO₂ | - | 35 | 17 |
| Example 3-7 | nano-FeₓP/TiO₂ | MgO | >99 | 96 |
| Example 3-8 | nano-FeₓP/TiO₂ | MgSO₄ | 97 | 89 |
| Example 3-9 | nano-FeₓP/TiO₂ | K₂CO₃ | 83 | 71 |
| Example 3-10 | nano-FeₓP/TiO₂ | Et₃N | 90 | 82 |
| Example 3-11 | nano-FeₓP/TiO₂ | DBU | 92 | 81 |
| Example 3-12 | nano-FeₓP/TiO₂ | DIPEA | 88 | 76 |
| Comparative Example 3-1 | FeOₓ/TiO₂ | - | 0 | 0 |
| Comparative Example 3-2 | Bulk- Fe₂P | - | 0 | 0 |
| Comparative Example 3-3 | Raney iron | - | 6 | 2 |

| | | | | |
|---|---|---|---|---|
| 1) Measurement was performed using an internal standard with gas chromatography (GC) (a gas chromatograph (trade name "GC-2014") equipped with InertCap for amines, manufactured by SHIMADZU CORPORATION). 2) The reaction time was changed from 2 hours to 6 hours. 3) As the reaction condition, H₂:NH₃=3.8 MPa:0.2 MPa was changed to H₂:NH₃=1 | | | | |

MPa:0.2 MPa, and the reaction time was changed from 2 hours to 12 hours.

In the table, Et₃N represents triethyl amine, DBU represents diazabicycloundecene, and DIPEA represents N,N-diisopropylethylamine.

As shown in Table 1, it was confirmed that the iron phosphide nanoparticles produced in Example 1-1 have catalytic activity as a hydrogenation catalyst. Different from iron nanoparticles whose surface is shielded, which is a conventional technology, the iron phosphide nanoparticles of the present invention have atmospheric stability and can maintain a low valence state at the particle surfaces as well, and thus, are considered to have catalytic activity. As shown in Table 1, the composite body of the present invention was significantly excellent in the catalytic activity as a hydrogenation catalyst. Further, as shown in Example 3-4, the composite body of the present invention was confirmed to be significantly excellent in sufficient catalytic activity even under a gentle condition in which the hydrogen pressure was reduced.

### [Evaluation of thermal stability]

An XAFS measurement on the iron phosphide nanoparticles according to Example 1-1 was performed under the following condition. The measurement result is shown in FIG. 5. In FIG. 5, the measurement results at 100°C and 150°C near 7110 eV completely overlapped with the result at room temperature, and were not able to be distinguished from each other. From FIG. 5, excellence in thermal stability was also confirmed.

### [Measurement condition]

Temperature increase and measurement were all performed in the atmosphere, using the Si (111) monochromator of SPring-8. The temperature increase rate was set to 10°C/min from room temperature (25°C), and the measurement times at respective temperatures were all 10 minutes. In FIG. 5, "r.t." represents room temperature.

The sample was measured at 25°C, the temperature was increased to 50°C, and when the temperature became stable, measurement was performed. Thereafter, every 50°C up to 500°C, temperature increase and measurement were repeated. After 500°C, temperature increase by 100°C and measurement were repeated, and measurement was performed up to 700°C.

### [Evaluation of durability]

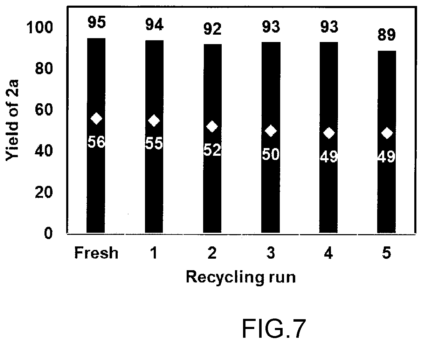

Durability as a catalyst was evaluated using the composite body of the iron phosphide nanoparticles and TiO₂ produced in Example 2-1. The composite body (nano-FeₓP/TiO₂) used in Example 3-2 was filtered to be recovered, and then, a reaction of producing benzylamine under the following reaction condition was repeated five times. The result is shown in FIG. 7.

### [Reaction condition]

benzonitrile (substrate): 0.5 mmol
nano-FeₓP/TiO₂ (catalyst): 0.1 g
2-propanol: 3 ml
H₂:NH₃=3.8 MPa:0.2 MPa
reaction time: 1 hour or 3 hours

In FIG. 7, the numerical value at a white diamond shape indicates the yield when the reaction time was 1 hour. In FIG. 7, for example, when the product was filtered to be recovered after Example 3-2, and then, used again in the reaction under the above condition with the reaction time set to 3 hours, the yield was 94% (yield after one cycle in FIG. 7).

From the result in FIG. 7, it was confirmed that the reduction catalyst of the present invention has high catalytic activity and is excellent in durability even when recovered and reused. Therefore, it can be said that the iron phosphide nanoparticles, the composite body, and the reduction catalyst of the present invention exhibit advantageous effects such as reducing waste and being able to be recovered to be reused, and the like.

### <ICP-AES of composite body>

With respect to the composite body of the iron phosphide nanoparticles and TiO₂ obtained in Example 2-1, observation was performed according to ICP-AES, using an ICP atomic emission spectroscopic analyzer (trade name "Optima 8300" manufactured by PerkinElmer, Inc., or the like). The result is shown in Table 2.

**[Table 2]**

| | wt% | |
|---|---|---|
| | Fe | P |
| nano-FeₓP/TiO₂ (immediately after production) | 2.01 | 1.04 |
| nano-FeₓP/TiO₂ (after use in hydrogenation reaction) | 1.96 | 0.98 |

In the observation result according to the ICP-AES, even after the use, the presence ratio between phosphorus atoms and iron atoms was scarcely changed. When both of the observation result according to the ICP-AES and the measurement result of the yield are taken into consideration, it is considered that the iron phosphide nanoparticles contained in the composite body maintain activity.

Further, with respect to the composite body before and after the use, electron microscopic observation was performed by the same method as that for the iron phosphide nanoparticles. The result is shown in FIG. 6. FIG. 6A shows a TEM image of the composite body before being used in the hydrogenation reaction, and FIG. 6B shows a TEM image of the composite body after being used once in the hydrogenation reaction. From comparison between the TEM images in FIG. 6A and FIG. 6B as well, change in morphology before and after the use was not observed.

### [Example 4: production of primary amine]

### <Example 4-1>

Benzonitrile was hydrogenated using the composite body (nano-FeₓP/TiO₂) produced in Example 2-1 as a catalyst, to produce benzylamine (2a in Table 5). Specifically, 0.1 g of the composite body (nano-FeₓP/TiO₂) (the amount of Fe was 0.036 mmol (the amount of Fe was 7.2 mol% with respect to 100 mol% of benzonitrile)) of Example 2-1, 0.1 mmol of magnesium oxide (MgO), 3 ml of 2-propanol, and 0.5 mmol of benzonitrile were put in an autoclave, with hydrogen gas and NH₃ gas pressurized so as to realize H₂:NH₃=3.8 MPa:0.2 MPa, and heated to 180°C, and reaction was carried out for 2 hours. The yield was 92%. Measurement of the yield was performed using an internal standard with gas chromatography (GC) (a gas chromatograph (trade name "GC-2014") equipped with InertCap for amines, manufactured by SHIMADZU CORPORATION).

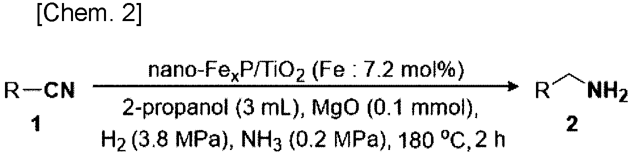

### <Examples 4-2 to 4-39>

A primary amine was produced by the same method as in Example 4-1 except that the type of the compound as the substrate that was used was changed to the nitrile compounds shown in Table 3 below. The measurement method for the yield was the same as that in Example 4-1. The correspondence relationship between the Example number and the obtained primary amine (primary amine in Table 5) is shown in Table 4. The obtained primary amine and the yield are shown in Table 5 below.

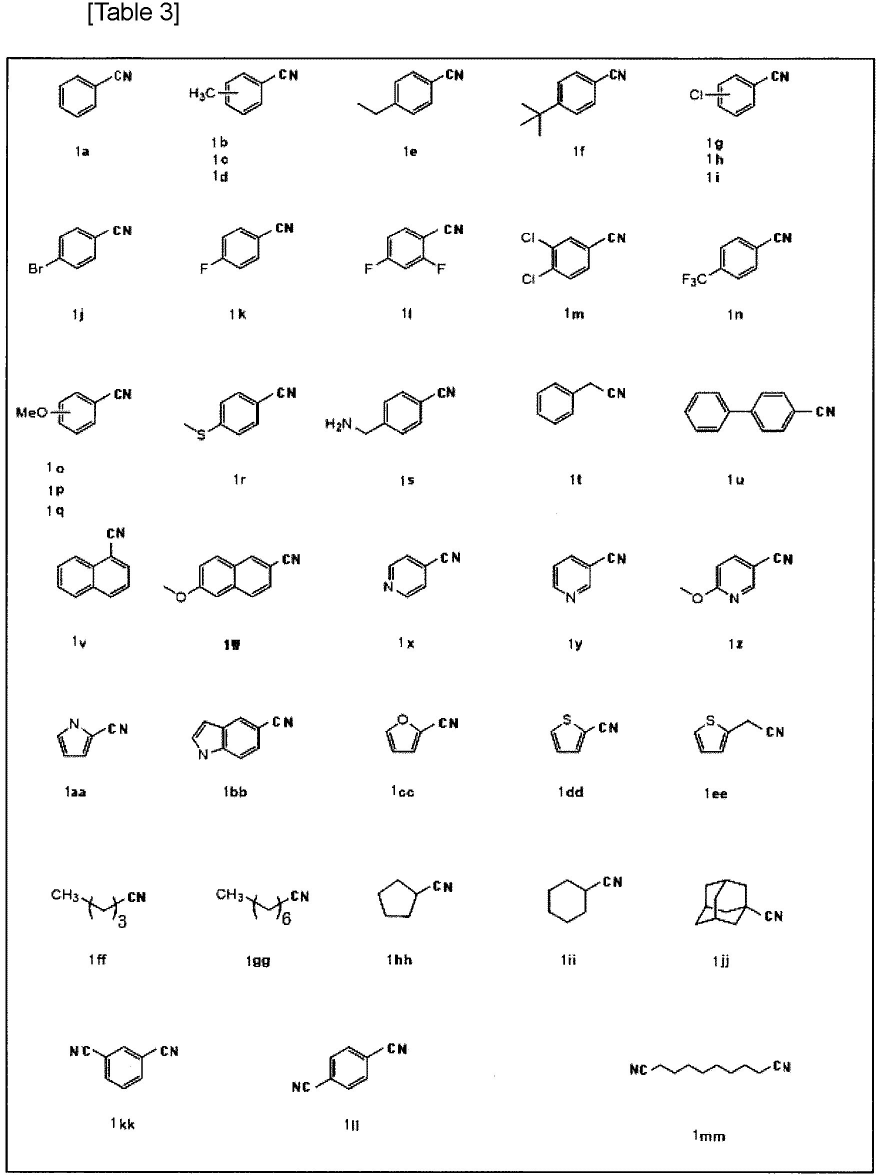

**[Table 4]**

| Example number | Primary amine in Table 5 | Example number | Primary amine in Table 5 | Example number | Primary amine in Table 5 |
|---|---|---|---|---|---|
| Example 4-1 | 2a | Example 4-16 | 2p | Example 4-31 | 2ee |
| Example 4-2 | 2b | Example 4-17 | 2q | Example 4-32 | 2ff |
| Example 4-3 | 2c | Example 4-18 | 2r | Example 4-33 | 2gg |
| Example 4-4 | 2d | Example 4-19 | 2s | Example 4-34 | 2hh |
| Example 4-5 | 2e | Example 4-20 | 2t | Example 4-35 | 2ii |
| Example 4-6 | 2f | Example 4-21 | 2u | Example 4-36 | 2jj |
| Example 4-7 | 2g | Example 4-22 | 2v | Example 4-37 | 2kk |
| Example 4-8 | 2h | Example 4-23 | 2w | Example 4-38 | 2ll |
| Example 4-9 | 2i | Example 4-24 | 2x | Example 4-39 | 2mm |
| Example 4-10 | 2j | Example 4-25 | 2y | | |
| Example 4-11 | 2k | Example 4-26 | 2z | | |
| Example 4-12 | 2l | Example 4-27 | 2aa | | |
| Example 4-13 | 2m | Example 4-28 | 2bb | | |
| Example 4-14 | 2n | Example 4-29 | 2cc | | |
| Example 4-15 | 2o | Example 4-30 | 2dd | | |

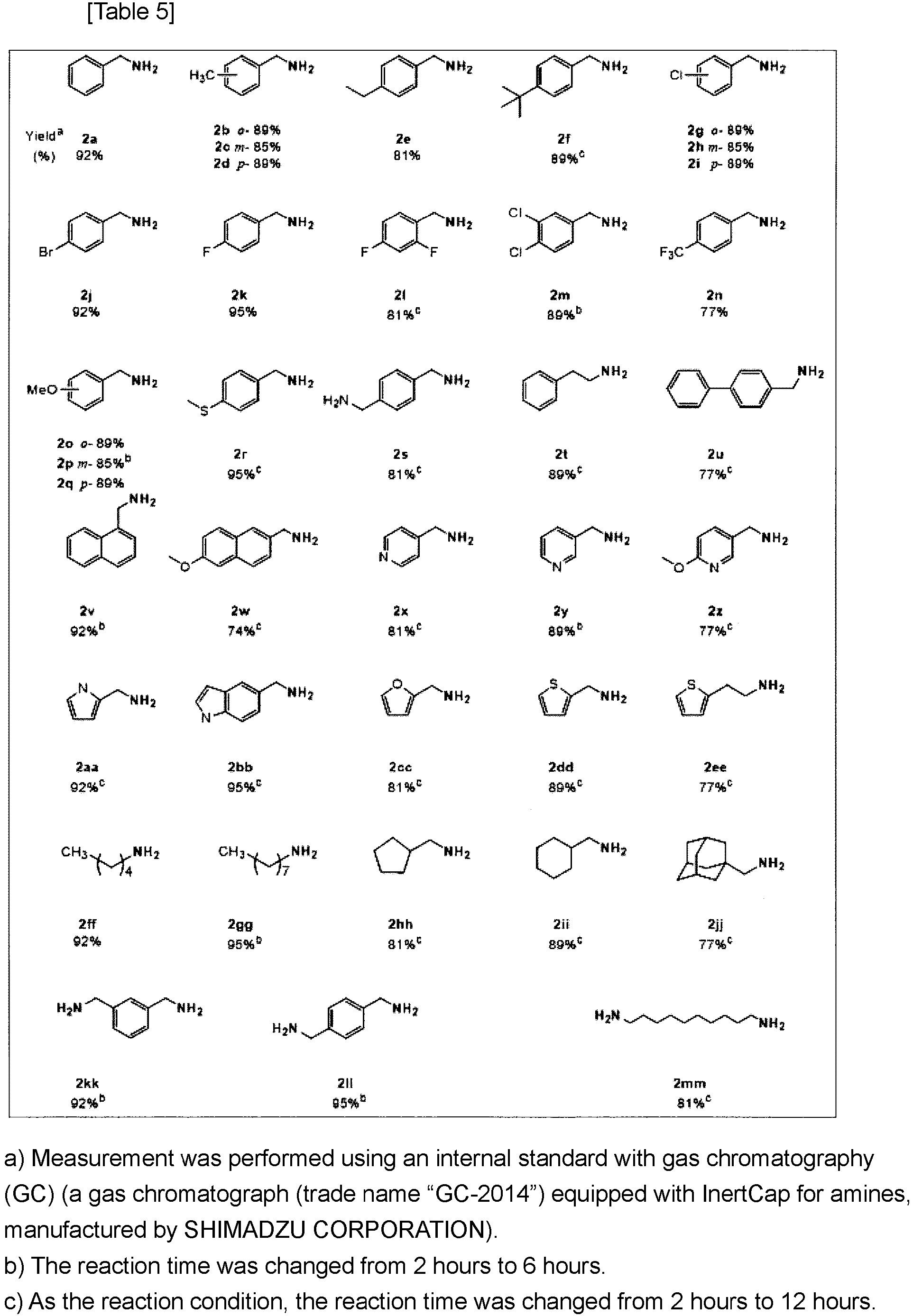

From the above result, it was able to be confirmed that the reduction catalyst (e.g., hydrogenation catalyst) of the present invention widely has catalytic activity with respect to many substrates and is excellent for a variety of substrates.

### INDUSTRIAL APPLICABILITY

The iron phosphide nanoparticles and the composite body of the present invention are useful as a reduction catalyst in reduction reactions (e.g., hydrogenation reaction), and particularly useful as a hydrogenation catalyst in production methods for amine compounds.

## Claims

1. Iron phosphide nanoparticles having peaks at diffraction angles (2θ±0.5°) of 48.3° and 32.7° in a powder X-ray diffraction measurement using CuKα radiation, wherein
when the iron phosphide nanoparticles are measured by X-ray photoelectron spectroscopy (XPS), iron atoms contained therein have a peak in a range of 706.0 to 707.5 eV in an Fe2p_{3/2} spectrum.

2. The iron phosphide nanoparticles according to claim 1, further having peaks at 40.2°, 52.9°, and 54.6° in the powder X-ray diffraction measurement.

3. The iron phosphide nanoparticles according to claim 1 or 2, further having a peak at 46.3° in the powder X-ray diffraction measurement.

4. The iron phosphide nanoparticles according to claim 1 or 2, wherein the iron phosphide nanoparticles are each a rod-shaped particle, and a maximum length in a major axis direction of the rod-shaped particle is less than 100 nm.

5. The iron phosphide nanoparticles according to claim 1 or 2, wherein a presence ratio between phosphorus atoms and iron atoms according to a scanning transmission electron microscope (STEM)-energy-dispersive X-ray spectroscopy (EDX) composition analysis is P:Fe=20%:80% to 80%:20%.

6. A composite body containing the iron phosphide nanoparticles according to claim 1 or 2 and a carrier.

7. The composite body according to claim 6, wherein the carrier is at least one type selected from the group consisting of a polymer, a chalcogen compound, a metal compound, a metal, and a solid carbon material.

8. A reduction catalyst containing the iron phosphide nanoparticles according to claim 1 or 2.

9. The reduction catalyst according to claim 8, further containing a carrier, wherein the iron phosphide nanoparticles and the carrier form a composite body.

10. A production method for a hydrogenated organic compound, wherein an organic compound is hydrogenated by using the reduction catalyst according to claim 8, to obtain the hydrogenated organic compound.

11. The production method for the hydrogenated organic compound according to claim 10, wherein the organic compound is a nitrile compound, the hydrogenated organic compound is a primary amine compound, and the nitrile compound is hydrogenated in a hydrogen atmosphere and in the presence of ammonia, at a hydrogen pressure of 8 MPa or less.

12. A production method for the iron phosphide nanoparticles according to claim 1 or 2, the production method comprising:
mixing a phosphorus compound and a surfactant under heating;
further heating an obtained mixture; and
further mixing an iron carbonyl compound thereto and heating a resultant matter, wherein
1-octadecene is not used.

13. The production method for the iron phosphide nanoparticles according to claim 12, wherein the phosphorous acid ester compound is a phosphite compound.

14. The production method for the iron phosphide nanoparticles according to claim 12, wherein the surfactant is an alkylamine.

15. The production method for the iron phosphide nanoparticles according to claim 12, wherein the iron carbonyl compound is at least one type selected from the group consisting of Fe(CO)s, Fe₂(CO)₉, and Fe₃(CO)₁₂.
